# EUROPEAN PATENT APPLICATION

(11) **EP 3 597 729 A1**
(43) Date of publication of application: **22.01.2020**
(21) Application number: 18774219.2
(22) Date of filing: 30.03.2018
(51) Int. Cl.: C12M 3/00, A61K 35/28, A61L 27/20, A61L 27/38, A61L 27/40, A61L 27/50, A61L 27/58, A61P 43/00, C12N 5/071

(54) **CELL CULTURING USING NANOFIBERS**

(30) Priority: 30.03.2017 JP 2017068377; 11.09.2017 JP 2017174237
(71) Applicant: Nissan Chemical Corporation, Tokyo 103-6119 (JP)
(72) Inventor: KANAKI, Tatsuro, Shiraoka-shi Saitama 349-0294 (JP); KIDA, Katsuhiko, Shiraoka-shi Saitama 349-0294 (JP); HAYASHI, Hisato, Funabashi-shi Chiba 274-0052 (JP); MINAMI, Masataka, Funabashi-shi Chiba 274-0052 (JP)
(74) Representative: J A Kemp LLP
(86) International application number: PCT/JP2018/013977
(87) International publication number: WO 2018/182016

(57) **Abstract**

The present invention provides a cell carrier containing a nanofiber composed of water-insoluble polysaccharides, preferably chitin or chitosan nanofiber; a carrier capable of being a common carrier in various operations such as i) suspension culture, ii) differentiation induction, iii) transportation and preservation under non-freezing conditions, iv) transplantation, v) recovery of bioactive substance from culture supernatant and the like of adherent cells; and continuous performance of plural operations selected from i) suspension culture, ii) differentiation induction, iii) transportation and preservation under non-freezing conditions, iv) transplantation, and v) recovery of bioactive substance from culture supernatant of adherent cells by using the carrier.

## Description

### [Technical Field]

The present invention relates to use of a nanofiber composed of water-insoluble polysaccharides as a carrier in culture, preservation, transportation or transplantation of cells.

### [Background Art]

In recent years, in the field of regenerative medicine, researches including culturing pluripotent stem cells such as iPS cells and ES cells to induce differentiation into intended organ cells-like ones and transplanting the obtained cells have been actively conducted. However, there is an ethical problem in the establishment of ES cells, and iPS cells have a problem of tumorigenicity risk. Also, it has been reported with regard to differentiation from iPS cells into cells constituting the intended organ that the final differentiation induction rate may be low and long-term culture may be required to obtain highly differentiated cells, thus revealing the cost problem. Given such problems, somatic stem cells such as mesenchymal stem cells and neural stem cells that have a low tumorigenicity risk, and precursor cells such as pre-adipocytes and progenitor cardiomyocytes that can shorten the differentiation induction period are attracting attention again. In particular, mesenchymal stem cells and pre-adipocytes, which are easily differentiated into fat, cartilage, and the like, have an advantage of easy availability and are used in various studies.

Furthermore, there is a high need for using, in addition to somatic stem cells and progenitor cells, primary cultured cells directly isolated from tissues. For example, primary hepatocytes prepared from human liver are seeded on a cell plate and transported, or stocked as frozen cells after sorting. Use of the obtained cells for tests greatly contributes to the study of drug metabolism of pharmaceutical products. In addition, primary cancer cells prepared by digesting cancer tissue isolated from cancer patients by enzyme treatment are used not only for cancer research but also for anticancer drug sensitivity tests and the like, and there is a great need.

In addition to mesenchymal stem cells isolated from bone marrow, mesenchymal stem cells include dental pulp-derived mesenchymal stem cells, adipose tissue-derived mesenchymal stem cells isolated together with adipocytes during liposuction and the like. These mesenchymal stem cells can be easily proliferated by single layer culture in a petri dish and further differentiated into fat, cartilage, and bone. Recently, it has also been clarified that these mesenchymal stem cells can differentiate into cells constituting the liver, pancreas and the like. Furthermore, consideration of not only transplantation of cells differentiated from mesenchymal stem cells but also transplantation of mesenchymal stem cells themselves has been started. More recently, cytokines and exosomes extracellularly secreted by the mesenchymal stem cells themselves have been focused on, and attempts to adhere mesenchymal stem cells to microcarriers, culture them, isolate physiologically active substances secreted in the culture supernatant, and apply them to pharmaceutical products and cosmetics have also been considered.

Pre-adipocytes are cells isolated together with adipocytes during liposuction, and can be easily separated in large amounts from a living body, like the adipose tissue-derived mesenchymal stem cells described above. Pre-adipocytes also have the property of being able to differentiate into not only adipocytes but also chondrocytes and the like, and application to not only adipocyte transplantation but also joint cartilage transplantation has been considered. In addition, it has been reported that the culture supernatant of adipocytes contains a component having a protection effect on the skin, and the use of a bioactive substance isolated from the culture supernatant for pharmaceutical products and cosmetics is also under consideration. Adipocytes are less likely to become cancer due to their low proliferative potency and advantageously permit transplantation to sites that are easy to transplant, such as subcutaneous tissue and the like. Thus, by subcutaneously transplanting adipocytes with a normal gene introduced therein to patients having a gene mutation, a disease caused by the gene mutation can be treated.

Under such circumstances, various problems are present in each step when conducting researches on mesenchymal stem cells and pre-adipocytes, applying transplantation of the cells for treatment, isolation of bioactive substances from the culture supernatant of the cells and the like. At present, mesenchymal stem cells, pre-adipocytes and the like are generally prepared in large amounts from one individual, frozen and the cryopreserved cells are used for various purposes. However, in a cell bank system expected in the future that manages the autologous transplantation system and manages and stores a large number of allogeneic cell stocks, it is difficult to stably supply a large amount of cells in a state suitable for transplantation by the current transportation technique that presupposes freezing of cells. That is, for transplantation, cryopreserved cells need to be thawed, cultured, and differentiated to the intended cells as necessary, and a large number of the cells in good condition need to be provided. When the cells need to be transported in a frozen state, the transplantation facility needs to obtain frozen cells from the cell bank that stores and manages the cells, and perform large-scale culture in the facility itself. For this end, it is necessary for the transplantation facility to have a large-scale cell culture equipment (e.g., cell processing center (CPC)), and transplantation therapy becomes difficult in a facility that does not have the equipment. On the other hand, cell banks generally have technique and equipment for mass culture of cells. If a large amount of cells in good condition suitable for transplantation are prepared on the cell bank side, the obtained cells can be quickly supplied to the transplantation facility while maintaining the good condition, transplantation therapy can be performed even in a facility that does not have a large-scale cell culture facility. To solve this problem, a technique for transporting a large amount of cells while maintaining the good condition without freezing is indispensable.

Techniques for culturing and proliferating mesenchymal stem cells and the like while being adhered to a microcarrier, transplanting the obtained cells, and collecting bioactive substances released into the culture supernatant have been studied. However, currently available microcarriers form sediment in the culture medium under static conditions, and need to be stirred during culture. A problem has been pointed out that cell death occurs due to collision between carriers during the stirring. Furthermore, when cells are recovered from microcarriers, the adhesion between cells and between cells and substrate needs to be loosened using a protease such as trypsin. Cell damage and decreased survival due to the protease also pose problems. In addition, since generally available microcarriers are not biodegradable, to apply the cultured cells to transplantation, they need to be detached from the microcarriers and collected. Furthermore, the cells need to be held by another biodegradable carrier on demand. These cell treatments are complicated and costly.

The present inventors have developed a medium composition for culturing animal and plant cells and/or tissues in a suspended state by using a nanofiber of polysaccharides and the like having enhanced dispersibility in water (patent document 1) .

### [Document List]

### [Patent documents]

patent document 1: WO 2015/111686 A1

### [SUMMARY OF THE INVENTION]

### [Problems to be Solved by the Invention]

The present invention aims to provide a technique for transporting a large amount of adherent cells such as mesenchymal stem cells, pre-adipocytes and the like under non-freezing conditions while maintaining a good condition. Also, the present invention aims to provide a new carrier that can solve various problems in conventional suspension culture of adherent cells using microcarriers such as cell death due to collision of microcarriers during stirring, cell damage due to protease treatment during passage, complicated cell treatments and the like required for subjecting the cells on microcarriers to transplantation and the like.

### [Means of Solving the Problems]

The present inventors have conducted intensive studies and found that the above-mentioned various problems can be solved at once by using, as a carrier, a nanofiber which is composed of polysaccharides such as chitin and the like, insoluble in a medium, suspended in the medium, has the activity to adhere cells, is biodegradable, and can be enzymatically digested without requiring protease. By mixing a nanofiber composed of polysaccharides in a liquid medium and culturing adherent cells such as mesenchymal stem cells and pre-adipocytes while being adhered to the nanofibers, the cells could be suspension cultured in a stationary state. The cells adhered to the nanofibers showed long-term survivability under suspension culture conditions. Mesenchymal stem cells and pre-adipocytes proliferated well on the nanofibers while maintaining their characters. When cultured in a differentiation induction medium, the pre-adipocytes adhered onto the nanofibers differentiated into adipocytes. Thus, it was also found that the cells on the nanofibers can be induced to differentiate. The cells adhered onto the nanofibers could be recovered by treating with a degrading enzyme of polysaccharides (e.g., chitin) constituting the nanofibers, without using protease and while minimizing damage to the cell. Since nanofibers composed of polysaccharides are biodegradable, the cells adhered to the nanofibers and still in the adhesion state could be formed into a sheet form, which is easy to transplant, together with the nanofibers. Since the cells adhered to the nanofibers easily form sediment by a centrifugation operation and the like, the culture supernatant could be easily recovered from the culture of the cells adhered to the nanofibers. Thus, it was found that a nanofiber composed of polysaccharides is useful as a carrier for cell transport under non-freezing conditions, culture, differentiation induction, transplantation and bioactive substance production, which are required for mesenchymal stem cells, pre-adipocytes, primary cells, and the like, and that using this carrier, a plurality of operations selected from the group consisting of cell culture, differentiation induction, cell transport, transplantation and bioactive substance production can be performed continuously.

Based on the above findings, they have conducted further studies and completed the present invention.

That is, the present invention is as follows:
[1] A carrier comprising a nanofiber composed of water-insoluble polysaccharides, for continuously subjecting adherent cells to two or more treatments selected from the group consisting of the following (1) to (3):
   (1) suspension culture,
   (2) preservation and/or transportation under non-freezing conditions, and
   (3) transplantation.
[2] The carrier of [1] wherein the suspension culture is for maintenance or proliferation of adherent cells, differentiation induction of adherent cells and/or production of humoral factor by adherent cells.
[3] The carrier of [1] or [2] wherein the carrier is for continuously subjecting the adherent cells to the treatments (1) to (3).
[4] The carrier of any of [1] to [3] wherein the adherent cell is a stem cell or a progenitor cell.
[5] The carrier of any of [1] to [3] wherein the adherent cell is a mesenchymal stem cell or a pre-adipocyte.
[6] The carrier of any of [1] to [5] wherein the water-insoluble polysaccharide is chitin or chitosan.
[7] A method for treating an adherent cell comprising continuously subjecting the adherent cell to two or more treatments selected from the group consisting of the following (1) to (3) while being attached to a nanofiber composed of water-insoluble polysaccharides:
   (1) suspension culture,
   (2) preservation and/or transportation under non-freezing conditions, and
   (3) transplantation.
[8] The method of [7] wherein the suspension culture is for maintenance or proliferation of adherent cells, differentiation induction of adherent cells and/or production of humoral factor by adherent cells.
[9] The method of [7] or [8] wherein the adherent cells are continuously subjected to the treatments (1) to (3).
[10] The method of any of [7] to [9] wherein the adherent cell is a stem cell or a progenitor cell.
[11] The method of any of [7] to [9] wherein the adherent cell is a mesenchymal stem cell or a pre-adipocyte.
[12] The method of any of [7] to [11] wherein the water-insoluble polysaccharide is chitin or chitosan.
[13] The method of any of [7] to [12] further comprising treating the adherent cells attached to the nanofiber composed of water-insoluble polysaccharides with a degrading enzyme of the water-insoluble polysaccharides to degrade the nanofiber, detaching the adherent cells from the nanofiber and recovering the adherent cells.
[14] A method for recovering an adherent cell attached to a nanofiber composed of water-insoluble polysaccharides comprising treating the adherent cell with a degrading enzyme of the water-insoluble polysaccharides to degrade the nanofiber, detaching the adherent cell from the nanofiber and recovering the adherent cell.
[15] The method of [14] wherein the water-insoluble polysaccharide is chitin or chitosan.
[16] The method of [15] wherein the degrading enzyme is chitinase, chitobiase, chitosanase or β-1,3-glucanase.

### [Effect of the Invention]

According to the present invention, adherent cells such as mesenchymal stem cells, pre-adipocytes and the like can be suspension cultured in a stationary state by culturing them while being attached to nanofibers composed of water-insoluble polysaccharides, without an operation such as shaking, rotation and the like having a risk of causing injury and loss of functions of cells. Since suspension culture of adherent cells (three-dimensional (3D) culture) is possible, a larger number of cells can be cultured with a smaller amount of medium compared to a single layer culture (two-dimensional (2D) culture).

In the culture using the medium composition of the present invention, maintenance culture of adherent cells such as mesenchymal stem cells, pre-adipocytes and the like can be performed or they may also be proliferated while maintaining the character thereof. Alternatively, undifferentiated cells such as mesenchymal stem cells, pre-adipocytes and the like can be differentiated into particular cells by culturing under particular differentiation conditions.

In the culture using the medium composition of the present invention, since suspension culture of the cells adhered to nanofibers composed of water-insoluble polysaccharides is performed, the cells adhered to the nanofibers can be easily removed by a centrifugation operation and the like and the culture supernatant can be recovered. As mentioned above, since a large number of cells can be cultured with a small amount of medium in suspension culture compared with single layer culture, useful bioactive substances released in the culture supernatant by the cells can be accumulated at high concentrations. Therefore, the medium composition of the present invention is useful for recovering and purifying useful bioactive substances released by adherent cells such as mesenchymal stem cells, pre-adipocytes and the like into the culture medium.

In addition, in the culture using the medium composition of the present invention, cells adhered on the nanofibers can be recovered by treating with a degrading enzyme (e.g., chitinase) of polysaccharides (e.g., chitin) constituting the nanofibers, without using animal-derived protease. Therefore, in the present invention, a passage operation of adherent cells such as mesenchymal stem cells, pre-adipocytes and the like can be performed, or the cells attached to the nanofibers can be detached from the microcarriers and collected while minimizing the damage to the cells due to protease, the risk of infection caused by contamination with animal components and the like.

Adherent cells such as mesenchymal stem cells, pre-adipocytes and the like show good survivability for a long term while being adhered to nanofibers composed of water-insoluble polysaccharides. Therefore, the medium composition of the present invention is useful for storing the aforementioned adherent cells and transporting same under non-freezing conditions. For example, when adhesion culture of cells is performed on a plate, and the plate is directly transported, the cells may be detached from the plate due to trembling during transport, thus degrading the inherent function of the cells. In the medium composition of the present invention, however, the cells adhered to the nanofibers can be maintained in a suspended state, and therefore, damage on the cells resulting from trembling during transport that detaches the cells from the plate and the like can be avoided, and the cells can be preserved and transported under non-freezing conditions while maintaining the inherent functions thereof. In addition, a large number of cells can be preserved and transported with a small amount of medium and space compared with preservation and transportation in the state of single layer culture.

Since nanofibers composed of water-insoluble polysaccharides are biodegradable, the cells adhered to the nanofibers can be transplanted in an adhesion state to a living body together with the nanofibers. Therefore, adherent cells such as mesenchymal stem cells, pre-adipocytes and the like are cultured in the medium composition of the present invention, differentiated to a desired differentiation phase and the obtained cells can be transplanted to a living body together with the nanofibers without detaching from the nanofibers. Therefore, the risk of cell damage and loss of function due to the cell detaching operation can be avoided, and the cells in a good condition can be subjected to transplantation.

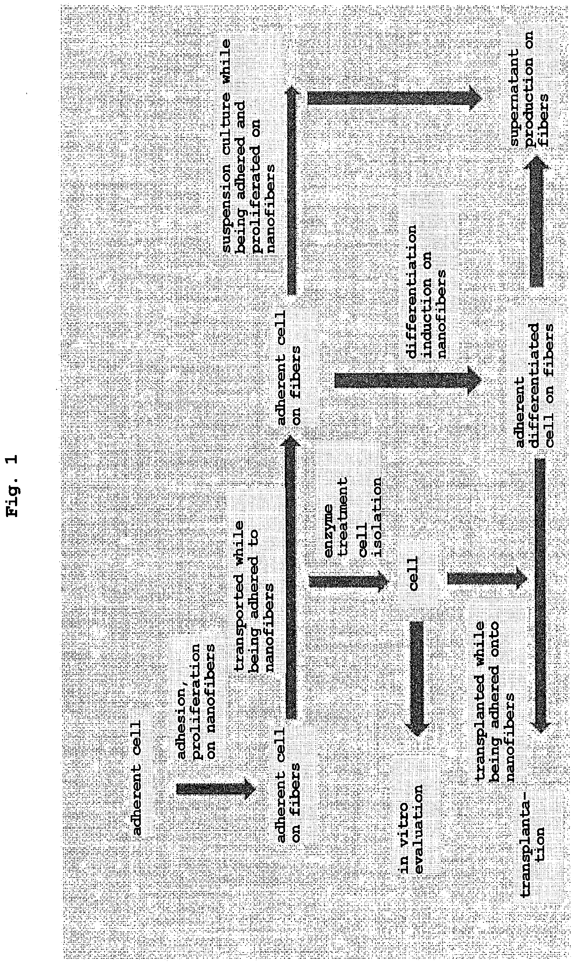

Thus, nanofibers composed of water-insoluble polysaccharides can be used as a common carrier in various operations such as i) culture, ii) differentiation induction, iii) transportation and preservation under non-freezing conditions, iv) transplantation, v) recovery of bioactive substance from culture supernatant and the like of adherent cells such as mesenchymal stem cells, pre-adipocytes and the like. Using the carrier, therefore, plural operations selected from the aforementioned i) culture, ii) differentiation induction, iii) transportation and preservation under non-freezing conditions, iv) transplantation, v) recovery of bioactive substance from culture supernatant can be performed continuously (Fig. 1).

### [Brief Description of the Drawings]

Fig. 1 is a schematic showing of one embodiment in which adherent cells are continuously subjected to plural operations of i) culture, ii) differentiation induction, iii) transportation and preservation under non-freezing conditions, iv) transplantation, v) recovery of bioactive substance from culture supernatant and the like by using a nanofiber composed of water-insoluble polysaccharides as a common carrier.
Fig. 2 is a photograph of human pre-adipocytes dispersed in single cells by a Yatalase treatment.
Fig. 3 shows migration activity and proliferation capacity of human pre-adipocytes maintained on a chitin nanofiber at 37°C or 25°C for 7 days.
Fig. 4 shows adipocytes differentiated from human pre-adipocytes maintained on a chitin nanofiber at 37°C or 25°C for 7 days.
Fig. 5 shows migration activity and proliferation capacity of human adipose-derived mesenchymal stem cells maintained on a chitin nanofiber at 37°C or 25°C for 7 days.
Fig. 6 shows adipocytes differentiated from human adipose-derived mesenchymal stem cells maintained on a chitin nanofiber at 37°C or 25°C for 7 days.
Fig. 7 shows a conceptual diagram of a cell transport method using a chitin nanofiber.
Fig. 8 shows the state of a human pre-adipocyte sheet produced by culturing on a chitin nanofiber at 37°C or 25°C for 10 days.

### [Description of Embodiments]

The present invention provides use of a nanofiber composed of water-insoluble polysaccharides as a carrier in various culture methodologies for adherent cells. Adherent cells can be subjected to various treatments such as (1) suspension culture, (2) preservation and/or transportation under non-freezing conditions, (3) transplantation and the like while being attached to a nanofiber composed of water-insoluble polysaccharides. Therefore, the present invention can also be comprehended as a method for treating adherent cells including subjecting the adherent cells to a treatment such as (1) suspension culture, (2) preservation and/or transportation under non-freezing conditions, (3) transplantation and the like while being attached to a nanofiber composed of water-insoluble polysaccharides.

### [adherent cells]

Cell is a most basic unit constituting animals and plants, which has, as its elements, cytoplasm and various organelles inside the cellular membrane. In this case, the nucleus encapsulating the DNA may or may not be contained intracellularly.

Examples of the animal include insect, fish, amphibian, reptiles, birds, pancrustacea, hexapoda, mammals and the like, with preference given to mammal. Examples of the mammal include, but are not limited to, rat, mouse, rabbit, guinea pig, squirrel, hamster, vole, platypus, dolphin, whale, dog, cat, goat, bovine, horse, sheep, swine, elephant, common marmoset, squirrel monkey, Macaca mulatta, chimpanzee, human and the like. The plant is not particularly limited as long as the collected cells can be applied to liquid culture. Examples thereof include, but are not limited to, plants (e.g., ginseng, periwinkle, henbane, coptis, belladonna etc.) producing crude drugs (e.g., saponin, alkaloids, berberine, scopolin, phytosterol etc.), plants (e.g., blueberry, safflower, madder, saffron etc.) producing dye or polysaccharide (e.g., anthocyanin, safflower dye, madder dye, saffron dye, flavones etc.) to be a starting material for cosmetic or food, or plants producing a pharmaceutical active pharmaceutical ingredient, and the like. In the present invention, mammalian cells are preferably used.

In one embodiment of the present invention, adherent cells are used. Adherent cell is a cell that requires a scaffold such as a container wall and the like for survival and proliferation. In the present invention, since adherent cells attach to nanofibers composed of water-insoluble polysaccharides, treatments such as good suspension culture, preservation and/or transportation under non-freezing conditions, transplantation and the like of the cells are achieved.

The adherent cell to be used in the present invention is not particularly limited and, for example, stem cell, progenitor cell, somatic non-stem cell, primary cultured cell, cell line, cancer cell and the like can be mentioned. Stem cell is a cell concurrently having an ability to replicate itself, and an ability to differentiate into other plural lineages. Examples of the adherent stem cell include, but are not limited to, somatic stem cell and the like such as mesenchymal stem cell, neural stem cell, hematopoietic stem cell, liver stem cell, pancreas stem cell, muscle stem cell, reproductive stem cell, intestinal stem cell, cancer stem cell, hair follicle stem cell and the like. Mesenchymal stem cell is a stem cell having differentiation potency into all or some of osteocyte, chondrocyte and adipocyte. Mesenchymal stem cell is present in a tissue such as bone marrow, peripheral blood, cord blood, adipose tissue and the like at a low frequency and can be isolated from these tissues by a known method. Progenitor cell is a cell on the way to differentiate from the aforementioned stem cell into a particular somatic cell or reproductive cell. Examples of the adherent progenitor cell include, but are not limited to, pre-adipocyte, cardiac muscle progenitor cell, endothelial progenitor cell, neural progenitor cell, liver progenitor cell, pancreas progenitor cell, kidney progenitor cell and the like. Examples of the adherent somatic non-stem cell include, but are not limited to, fibroblast, osteocyte, bone pericyte, keratinocyte, adipocyte, mesenchymal cell, epithelial cell, epidermal cell, endothelial cell, vascular endothelial cell, hepatocyte, chondrocyte, cumulus cell, neural cell, glial cell, neuron, oligodendrocyte, microglia, astrocyte, heartcell, esophagus cell, muscle cell (e.g., smooth muscle cell or skeletal muscle cell), pancreas beta cell, melanin cell, and the like. Primary cultured cell is a cell after separation of cells and tissues from a living body and in a state of culture before performing the first passage. The primary cultured cell may be a cell collected from any tissue, for example, skin, kidney, spleen, adrenal gland, liver, lung, ovary, pancreas, uterus, stomach, colon, small intestine, large intestine, spleen, bladder, prostate, testis, thymus, muscle, bond tissue, bone, joints, blood vessel tissue, blood, heart, eye, brain, nerve tissue and the like. Cell lines are cells that have acquired infinite proliferative capacity by an artificial operation in vitro. The adherent cell to be used in the present invention is preferably a stem cell or progenitor cell, more preferably a mesenchymal stem cell or pre-adipocyte.

### [nanofiber]

The nanofibers to be used in the present invention are dispersed in a liquid medium and show an effect of suspending the cells (preferably, adherent cells) attached to the nanofiber in the liquid medium.

In the present specification, nanofiber refers to a fiber having an average fiber diameter (D) of 0.001 to 1.00 µm. The average fiber diameter of the nanofiber to be used in the present invention is preferably 0.005 to 0.50 µm, more preferably 0.01 to 0.05 µm, further preferably 0.01 to 0.02 µm.

The aspect ratio (L/D) of the nanofiber to be used in the present invention is not particularly limited and is obtained from average fiber length/average fiber diameter, and is generally 2 - 500, preferably 5 - 300, more preferably 10 - 250.

In the present specification, the average fiber diameter (D) of the nanofiber is determined as follows. First, a hydrophilizing treatment of a collodion support film manufactured by Okenshoji Co., Ltd. is performed for 3 min by an ion cleaner (JIC-410) manufactured by JEOL Ltd., several drops of a nanofiber dispersion (diluted with ultrapure water) to be the evaluation target is added dropwise, and dried at room temperature. This is observed under a transmission electron microscope (TEM, H-8000) (10,000-fold) manufactured by Hitachi, Ltd. at an accelerating voltage 200 kV. Using the obtained image, the fiber diameter of each one of the nanofibers (specimen number: 200 - 250) is measured, and the mean thereof is taken as the average fiber diameter (D).

In addition, the average fiber length (L) is determined as follows. A nanofiber dispersion to be the evaluation target is diluted to 100 ppm with pure water, and nanofibers are uniformly dispersed using an ultrasonic cleaner. The nanofiber dispersion is cast on a silicon wafer subjected in advance to a hydrophilizing treatment of the surface with conc. sulfuric acid, dried at 110°C for 1 hr and used as a sample. Using an image obtained by observing the obtained sample under a scanning electron microscope (SEM, JSM-7400F) (2,000-fold), the fiber length of each one of the nanofibers (specimen number: 150 - 250) is measured, and the mean thereof is taken as the average fiber length (L).

In a preferable embodiment, the nanofiber to be used in the present invention is, upon mixing with a liquid medium, uniformly dispersed in the liquid while maintaining the primary fiber diameter, substantially retains the cells attached to the nanofiber without substantially increasing the viscosity of the liquid, and shows an effect of preventing sediment thereof. Without substantially increasing the viscosity of the liquid means that the viscosity of the liquid does not exceed 8 mPa·s. In this case, the viscosity of the liquid (that is, the viscosity of the following medium composition of the present invention) is not more than 8 mPa·s, preferably not more than 4 mPa·s, more preferably not more than 2 mPa·s. The viscosity of the liquid containing the nanofiber can be measured, for example, using a tuning fork vibration type viscometer (SV-1A, A&D Company Ltd.) under 25°C conditions.

The nanofiber to be used in the present invention is constituted of water-insoluble polysaccharides. Saccharides mean glycopolymers wherein not less than 10 single saccharides (e.g., triose, tetrose, pentose, hexsauce, heptose etc.) are polymerized.

Examples of the water-insoluble polysaccharides include, but are not limited to, celluloses such as cellulose, hemicellulose and the like; chitinous substances such as chitin, chitosan and the like, and the like. The water-insoluble polysaccharides are preferably chitin or chitosan, more preferably chitin.

Cellulose is a natural polymer compound wherein D-glucopyranoses which are a 6-membered ring of glucose, are β-1, 4 glucoside bonded. As the starting material, for example, plant-derived cellulose such as lumber, bamboo, hemp, jute, kenaf, cotton, agricultural crops_{•}food residue and the like, or cellulose of microorganism production or animal production such as bacterial cellulose, Cladophora, Glaucocystis, Valonia, Tunicate cellulose and the like can be used. In the plant-derived celluloses, very fine fibers called microfibrils are bundled to form higher structures in stages such as fibril, lamella and fibre cell. In addition, in bacterial cellulose, cellulose microfibrils secreted from bacterial cells and having the unchanged thickness form a fine net structure.

In the present invention, cellulose material having high purity such as cotton, bacterial cellulose and the like can be directly used. However, other plant-derived cellulose and the like are preferably used after isolation and purification. Cellulose preferably used in the present invention includes cotton cellulose, bacterial cellulose, kraft pulp cellulose, crystalline cellulose and the like.

The chitinous substance refers to one or more carbohydrates selected from the group consisting of chitin and chitosan. Major sugar units constituting chitin and chitosan are N-acetylglucosamine and glucosamine, respectively. Generally, chitin has a high N-acetylglucosamine content and is poorly soluble in acidic aqueous solution, and chitosan has a high glucosamine content and is soluble in acidic aqueous solution. For convenience, chitin contains not less than 50% of N-acetylglucosamine in the constituent sugar, and chitosan contains less than 50% of N-acetylglucosamine in the present specification. To achieve a high suspending action, a higher ratio of N-acetylglucosamine in the sugar unit constituting chitin is more preferable. The ratio of N-acetylglucosamine in the sugar unit constituting chitin is preferably not less than 80%, more preferably not less than 90%, further preferably not less than 98%, most preferably 100%.

As the starting material of chitin, many biological resources such as shrimps, crabs, insect, shells, mushrooms and the like can be used. The chitin to be used in the present invention may be one having α-form crystal structure such as chitin derived from crab shell, shrimp shell and the like, or one having β-form crystal structure such as chitin derived from cuttlebones and the like. The test of crabs and shrimps is often regarded as industrial waste and preferable as a starting material since it is easily available and effectively used. On the other hand, it requires a protein removing step and a decalcification step to remove protein, minerals and the like contained as impurities. In the present invention, therefore, purified chitin that underwent a matrix removal treatment is preferably used. Purified chitin is commercially available.

By pulverizing the starting material containing the aforementioned water-insoluble polysaccharides, a nanofiber constituted of the water-insoluble polysaccharides can be obtained. While the pulverization method is not limited, a method affording a strong shear force such as a medium stirring mill, for example, a high-pressure homogenizer, a grinder (stone mill), a bead mill and the like is preferable for subdivision to the below-mentioned fiber diameter and fiber length meeting the object of the present invention.

Of these, subdivision by a high-pressure homogenizer is preferable, and, for example, subdivision (pulverization) by the wet grinding method disclosed in JP-A-2005-270891 or JP-B-5232976 is desirable. Specifically, the starting material is pulverized by spraying a dispersion of a starting material from a pair of nozzles at a high-pressure and bombarding each other, and, for example, Star Burst system (high-pressure pulverization device manufactured by Sugino Machine Limited) or NanoVater (high-pressure pulverization device of yoshida kikai co., ltd.) is used therefor.

In the subdivision (pulverization) of a starting material by the aforementioned high-pressure homogenizer, the degree of subdivision and homogenization depends on the pressure in pumping into an ultrahigh-pressure chamber in a high-pressure homogenizer, and the number (treatment number) of passage through the ultrahigh-pressure chamber, and the concentration of the starting material in the water dispersion. The pumping pressure (treatment pressure) is not particularly limited and it is generally 50 - 250 MPa, preferably 150 - 245 MPa.

While the concentration of the starting material in a water dispersion during the subdividing treatment is not particularly limited, it is generally 0.1 mass % - 30 mass %, preferably 1 mass % - 10 mass %. While the treatment number of the subdivision (pulverization) is not particularly limited, it varies depending on the concentration of the starting material in the aforementioned water dispersion. When the concentration of the starting material is 0.1 - 1 mass %, the treatment number of 10 - 100 is sufficient for pulverization, but 1 - 10 mass % sometimes requires about 10 - 1000 times of treatment.

The viscosity of the water dispersion during the aforementioned subdivision treatment is not particularly limited. For example, when the water-insoluble polysaccharide is α chitin, the viscosity of the water dispersion is within the range of 1 - 100 mPa·S, preferably 1 - 85 mPa·S (by tuning fork vibration type viscometer (SV-1A, A&D Company Ltd.) under 25°C conditions). The particle size of the water-insoluble polysaccharides in a water dispersion during a subdivision treatment is not particularly limited. For example, when the water-insoluble polysaccharide is α chitin, the average particle size of α chitin in the water dispersion is within the range of 0.5 - 200 µm, preferably 30 - 150 µm (by laser diffraction/scattering type particle size distribution measurement apparatus LA-960 (Horiba, Ltd.)).

The preparation method of a nanofiber is described in WO 2015/111686 A1 and the likes.

### [Medium composition containing nanofiber]

In the present invention, when adherent cells attached to a nanofiber composed of water-insoluble polysaccharides are subjected to suspension culture or preservation and/or transportation under non-freezing condition, a liquid medium composition containing the nanofiber (to be also referred to as the medium composition of the present invention in the present specification) is used.

In a preferable embodiment, nanofibers composed of water-insoluble polysaccharides are uniformly dispersed in the medium composition of the present invention, and adherent cells attached to the nanofibers are suspended in the liquid medium.

Suspending of cells in the present invention refers to a state where cells do not adhere to a culture container (non-adhesive). Furthermore, in the present invention, when the cells are proliferated, differentiated or maintained, the state where the cells and/or tissues are uniformly dispersed and suspended in the liquid medium composition in the absence of a pressure on or vibration of the liquid medium composition from the outside or shaking, rotating operation and the like in the composition is referred to as "suspension standing", and cultivation of the cells and/or tissues in such condition is referred to as "suspension standing culture". In the "suspension standing", the period of suspending includes at least 5 min, preferably, not less than 1 hr, not less than 24 hr, not less than 48 hr, not less than 6 days, not less than 21 days, though the period is not limited thereto as long as the suspended state is maintained.

In a preferable embodiment, the medium composition of the present invention permits suspension standing of cells at least on one point in the temperature range (e.g., 0 - 40°C) capable of culturing, preserving or transporting cells. The medium composition of the present invention permits suspension standing of cells at least on one point in the temperature range of preferably 25 - 37°C, most preferably 37°C.

Whether or not suspension standing is possible can be evaluated by, for example, uniformly dispersing polystyrene beads (Size 500-600 µm, manufactured by Polysciences Inc.) in a medium composition to be the evaluation target, standing same at 25°C, and observing whether the suspended state of the cell can be maintained for at least 5 min (preferably, not less than 24 hr, not less than 48 hr).

The concentration of the nanofiber composed of water-insoluble polysaccharides in the medium composition of the present invention can be appropriately determined such that when adherent cells are subjected to suspension culture, preservation or tranportation under non-freezing condition by using the medium composition, the survivability of the adherent cells can be improved, or the adherent cells can be suspended (preferably suspension stood). For example, in the case of chitin nanofiber, the concentration of the chitin nanofiber in the medium composition is, for example, not less than 0.0001% (weight/volume), preferably not less than 0.001% (weight/volume). The upper limit of the concentration of the chitin nanofiber in the medium composition is, for example, not more than 1.0% (weight/volume), preferably not more than 0.1% (weight/volume).

The medium contained in the medium composition of the present invention can be appropriately selected according to the kind and the like of the adherent cells to be used. For example, when used for the purpose of culture, preservation or transportation of mammalian adherent cells, a medium generally used for culturing mammalian cells can be used as a medium to be contained in the medium composition of the present invention. Examples of the medium for mammalian cells include Dulbecco's Modified Eagle's Medium (DMEM), hamF12 medium (Ham's Nutrient Mixture F12), DMEM/F12 medium, McCoy's 5A medium, Eagle MEM medium (Eagle's Minimum Essential Medium; EMEM), αMEM medium (alpha Modified Eagle's Minimum Essential Medium; αMEM), MEM medium (Minimum Essential Medium), RPMI1640 medium, Iscove's Modified Dulbecco's Medium (IMDM), MCDB131 medium, William medium E, IPL41 medium, Fischer's medium, StemPro34 (manufactured by Invitrogen), X-VIVO 10 (manufactured by Cambrex Corporation), X-VIVO 15 (manufactured by Cambrex Corporation), HPGM (manufactured by Cambrex Corporation), StemSpan H3000 (manufactured by STEMCELL Technologies), StemSpanSFEM (manufactured by STEMCELL Technologies), StemlineII (manufactured by Sigma Aldrich), QBSF-60 (manufactured by Quality Biological), StemPro hESC SFM (manufactured by Invitrogen), mTeSR1 or 2 medium (manufactured by STEMCELL Technologies), Sf-900II (manufactured by Invitrogen), Opti-Pro (manufactured by Invitrogen), and the like.

Those of ordinary skill in the art can freely add, according to the object, sodium, potassium, calcium, magnesium, phosphorus, chlorine, various amino acids, various vitamins, antibiotic, serum, fatty acid, sugar and the like to the above-mentioned medium. For culture, preservation or transportation of mammalian cells, those of ordinary skill in the art can also add, according to the object, one or more kinds of other chemical components and biogenic substances in combination. Examples of the components to be added to a medium for mammalian cells include fetal bovine serum, human serum, horse serum, insulin, transferrin, lactoferrin, cholesterol, ethanolamine, sodium selenite, monothioglycerol, 2-mercaptoethanol, bovine serum albumin, sodium pyruvate, polyethylene glycol, various vitamins, various amino acids, agar, agarose, collagen, methylcellulose, various cytokines, various hormones, various proliferation factors, various extracellular matrices, various cell adhesion molecules and the like. Examples of the cytokine to be added to a medium include, but are not limited to, interleukin-1 (IL-1), interleukin-2 (IL-2), interleukin-3 (IL-3), interleukin-4 (IL-4), interleukin-5 (IL-5), interleukin-6 (IL-6), interleukin-7 (IL-7), interleukin-8 (IL-8), interleukin-9 (IL-9), interleukin-10 (IL-10), interleukin-11 (IL-11), interleukin-12 (IL-12), interleukin-13 (IL-13), interleukin-14 (IL-14), interleukin-15 (IL-15), interleukin-18 (IL-18), interleukin-21 (IL-21), interferon-α (IFN-α), interferon-β (IFN-β), interferon-γ (IFN-γ), granulocyte colony stimulating factor (G-CSF), monocyte colony stimulating factor (M-CSF), granulocyte-macrophage colony stimulating factor (GM-CSF), stem cell factor (SCF), flk2/flt3 ligand (FL), leukemia cell inhibitory factor (LIF), oncostatin M (OM), erythropoietin (EPO), thrombopoietin (TPO) and the like.

Examples of the hormone to be added to a medium include, but are not limited to, melatonin, serotonin, thyroxine, triiodothyronine, epinephrine, norepinephrine, dopamine, anti-Mullerian hormone, adiponectin, adrenocorticotropic hormone, angiotensinogen and angiotensin, antidiuretic hormone, atrial natriuretic peptide, calcitonin, cholecystokinin, corticotropin release hormone, erythropoietin, follicle stimulating hormone, gastrin, ghrelin, glucagon, gonadotropin release hormone, growth hormone release hormone, human chorionic gonadotropin, human placental lactogen, growth hormone, inhibin, insulin, insulin-like growth factor, leptin, luteinizing hormone, melanocyte stimulating hormone, oxytocin, parathyroid hormone, prolactin, gastrin-releasing peptide, somatostatin, thrombopoietin, thyroid-stimulating hormone, thyrotropin releasing hormone, cortisol, androstenedione, testosterone, dehydroepiandrosterone, androstenedione, dihydrotestosterone, estradiol, estrone, estriol, progesterone, calcitriol, calcidiol, prostaglandin, leukotriene, prostacyclin, thromboxane, prolactin releasing hormone, lipotropin, brain natriuretic peptide, neuropeptide Y, histamine, endothelin, pancreas polypeptide, rennin and enkephalin.

Examples of the growth factor to be added to a medium include, but are not limited to, transforming growth factor-α (TGF-α), transforming growth factor-β (TGF-β), macrophage inflammatory protein-1α (MIP-1α), epithelial cell growth factor (EGF), fibroblast growth factor-1, 2, 3, 4, 5, 6, 7, 8 or 9 (FGF-1, 2, 3, 4, 5, 6, 7, 8, 9), nerve cell growth factor (NGF) hepatocyte growth factor (HGF), leukemia inhibitory factor (LIF), protease nexin I, protease nexin II, platelet-derived growth factor (PDGF), choline vasoactive differentiation factor (CDF), chemokine, Notch ligand (Delta1 and the like), Wnt protein, angiopoietin-like protein 2, 3, 5 or 7 (Angpt2, 3, 5, 7), insulin like growth factor (IGF), insulin-like growth factor binding protein-1 (IGFBP), Pleiotrophin and the like.

In addition, these cytokines and growth factors having amino acid sequences artificially altered by gene recombinant techniques can also be added. Examples thereof include IL-6/soluble IL-6 receptor complex, Hyper IL-6 (fusion protein of IL-6 and soluble IL-6 receptor) and the like.

Examples of the various extracellular matrices and various cell adhesion molecules include collagen I to XIX, fibronectin, vitronectin, laminin-1 to 12, nitrogen, tenascin, thrombospondin, von Willebrand factor, osteopontin, fibrinogen, various elastins, various proteoglycans, various cadherins, desmocolin, desmoglein, various integrins, E-selectin, P-selectin, L-selectin, immunoglobulin superfamily, Matrigel, poly-D-lysine, poly-L-lysine, chitin, chitosan, sepharose, hyaluronic acid, alginate gel, various hydrogels, cleavage fragments thereof and the like.

Examples of the antibiotic to be added to a medium include Sulfonamides and preparations, penicillin, phenethicillin, methicillin, oxacillin, cloxacillin, dicloxacillin, flucloxacillin, nafcillin, ampicillin, penicillin, amoxicillin, ciclacillin, carbenicillin, ticarcillin, piperacillin, azlocillin, mezlocillin, mecillinam, andinocillin, cephalosporin and a derivative thereof, oxolinic acid, amifloxacin, temafloxacin, nalidixic acid, Piromidic acid, ciprofloxacin, cinoxacin, norfloxacin, perfloxacin, Rosaxacin, ofloxacin, enoxacin, pipemidic acid, sulbactam, clavulanic acid, β-bromopenisillanic acid, β-chloropenisillanic acid, 6-acetylmethylene-penisillanic acid, cephoxazole, sultampicillin, adinoshirin and sulbactam formaldehyde hudrate ester, tazobactam, aztreonam, sulfazethin, isosulfazethin, norcardicin, m-carboxyphenyl, phenylacetamidophosphonic acid methyl, Chlortetracycline, oxytetracycline, tetracycline, demeclocycline, doxycycline, methacycline, and minocycline.

The above-mentioned medium composition of the present invention can be produced by mixing the above-mentioned nanofibers composed of water-insoluble polysaccharides with an appropriate liquid medium such that the survivability of the adherent cells can be improved or a concentration at which the adherent cells can be suspended (preferably suspension standing) can be achieved, when the adherent cells are subjected to suspension culture, preservation or transportation under non-freezing conditions by using the medium composition.

In a preferable embodiment, a dispersion of the above-mentioned nanofiber composed of water-insoluble polysaccharides in a physiological aqueous solvent is mixed with a liquid medium to prepare the medium composition of the present invention. The dispersion may be sterilized (autoclave, gamma sterilization etc.). Alternatively, the dispersion and a liquid medium (aqueous solution as medium) prepared by dissolving the powder medium in water may be mixed, and used after sterilization. The dispersion and the liquid medium may be sterilized separately before mixing. Examples of the aqueous solvent include, but are not limited to, water, dimethyl sulfoxide (DMSO) and the like. As the aqueous solvent, water is preferable. The aqueous solvent may contain appropriate buffering agents and salts. The above-mentioned nanofiber dispersion is useful as a medium additive for preparing the medium composition of the present invention.

While the mixing ratio is not particularly limited, nanofiber dispersion:liquid medium (aqueous solution as medium) (volume ratio) is generally 1:99 - 99:1, preferably 10:90 - 90:10, more preferably, 20:80 - 80:20.

### [Treatment of adherent cell using nanofiber composed of water-insoluble polysaccharides]

The present invention provides use of a nanofiber composed of water-insoluble polysaccharides as a carrier in various culture methodologies for adherent cells. Adherent cells can be subjected to various treatments such as (1) suspension culture, (2) preservation and/or transportation under non-freezing conditions, (3) transplantation and the like while being attached to a nanofiber composed of water-insoluble polysaccharides. Therefore, the present invention can also be comprehended as a method for treating adherent cells including subjecting the adherent cells to a treatment such as (1) suspension culture, (2) preservation and/or transportation under non-freezing conditions, (3) transplantation and the like while being attached to a nanofiber composed of water-insoluble polysaccharides.

### (1) Suspension culture

Suspension culture of adherent cells can be performed by culturing adherent cells while being attached to a nanofiber composed of water-insoluble polysaccharides. The suspension culture can be performed by culturing adherent cells in the above-mentioned medium composition of the present invention. Nanofibers composed of water-insoluble polysaccharides show an effect of suspending the cells attached to the nanofibers in the medium (preferably effect of suspension standing). In the medium composition of the present invention, since nanofibers composed of water-insoluble polysaccharides are uniformly dispersed, when adherent cells are cultured in the medium composition, the adherent cells attach to the nanofibers and are suspended in the medium composition. By the suspending effect, a more increased amount of the number of cells per a given volume can be cultivated as compared to a single layer culture. In conventional suspension culture accompanying rotation or shaking operation, the proliferation rate and recovery rate of the cells may become low, or the function of the cell may be impaired since a shear force acts on the cells. Using the medium composition of the present invention, the cells can be cultured in a dispersion state without requiring an operation such as shaking and the like. Thus, easy suspension culture of a large amount of the object adherent cells without loss of the cell function can be expected. In addition, when cells are suspension cultured in a conventional medium containing a gel substrate, observation and recovery of the cells are sometimes difficult, and the function thereof is sometimes impaired during recovery. However, using the medium composition of the present invention, the cells under suspension culture are expected to be observed and recovered without impairing the function thereof. In addition, a conventional medium containing a gel substrate sometimes shows high viscosity that makes it difficult to exchange the medium. However, since the medium composition of the present invention has low viscosity, it is expected to be exchanged easily with a pipette, pump and the like.

When suspension culture of adherent cells is performed using a nanofiber composed of water-insoluble polysaccharides, adherent cells prepared separately are added to the culture composition of the present invention and mixed to give a uniform dispersion. In this case, the mixing method is not particularly limited and, for example, manual mixing using pipetting and the like, mixing using instrument such as stirrer, vortex mixer, microplate mixer, shaking machine and the like can be mentioned. After mixing, the obtained cell suspension may be cultured while being stood still, or cultured with rotation, shaking or stirring as necessary. The rotating speed and frequency can be appropriately set according to the object of those of ordinary skill in the art. For example, adherent cells are recovered from the passage culture, dispersed to a single cell or close thereto using an appropriate cell dissociation solution, the dispersed adherent cells are suspended in the medium composition of the present invention, and this is subjected to suspension culture (preferably, suspension standing culture).

The temperature when cells are cultivated is generally 25 to 39°C (e.g., 37°C), preferably 33 to 39°C, for animal cells. The CO₂ concentration is generally 4 to 10% by volume in the culture atmosphere, and 4 to 6% volume is preferable. The culture period may be set as appropriately according to the object of the culture.

When adherent cells are cultivated in the medium composition of the present invention, culture vessels generally used for cell culture such as schale, flask, plastic bag, Teflon (registered trade mark) bag, dish, schale, dish for tissue culture, multidish, microplate, microwell plate, multiplate, multiwell plate, chamber slide, tube, tray, culture bag, roller bottle and the like can be used for cultivation. These culture containers are desirably low cell-adhesive so that the adherent cells attached to a nanofiber will not adhere to the culture container. As a low cell-adhesive culture vessel, a culture vessel having a surface not artificially treated to improve adhesiveness to cells (e.g., coating treatment with extracellular matrix and the like), or a culture vessel having a surface artificially treated to reduce adhesiveness to cells can be used.

When the medium needs to be exchanged, the cells are separated by centrifugation or filtration treatment, and a fresh medium or the medium composition of the present invention can be added of the cells. Alternatively, the cells are appropriately concentrated by centrifugation or filtration treatment, and a fresh medium or the medium composition of the present invention can be added to the concentrated liquid. For example, unlimitatively, the gravitational acceleration (G) of centrifugation is 100G to 400G, and the size of the pore of the filter used for the filtration treatment is 10 µm to 100 µm.

The adherent cells can also be cultured by automatically conducting cell seeding, medium exchange, cell image obtainment, and recovery of cultured cells, under a mechanical control and under a closed environment while controlling pH, temperature, oxygen concentration and the like and using a bioreactor and an automatic incubator capable of high density culture.

### (1-1) Maintenance or proliferation of adherent cells

Since adherent cells are efficiently proliferated when they are subjected to suspension culture while being attached to a nanofiber composed of water-insoluble polysaccharides, the suspension culture is superior as a maintenance or proliferation method of the adherent cells. When adherent cells are subjected to suspension culture while being attached to a nanofiber composed of water-insoluble polysaccharides, they are dispersed while spreading three-dimensionally, without adhering to a culture container or without being locally present only on the bottom surface of the culture container, whereby the proliferation is promoted. Particularly, when chitin nanofiber is used as the nanofiber, the adherent cells attach to the chitin nanofiber, and strongly proliferate therefrom as a scaffold. As a result, the proliferated cells are connected like cluster of grapes on the nanofiber. This proliferation-promoting effect only requires presence of nanofibers at a concentration sufficient for suspending adherent cells and/or tissues (i.e., avoiding adhesion of adherent cells to culture container) in the medium composition, and capability of suspension standing (i.e., cells being uniformly dispersed and in a suspended state in liquid medium composition, without the presence of pressure, trembling, shaking, rotating operation and the like from the outside) is not essential. For example, in the case of chitin nanofiber, a proliferation-promoting effect is provided as long as a concentration of not less than 0.0001% (weight/volume), which is sufficient for expression of a suspending action, is achieved, even when the concentration is below 0.03% (weight/volume) which enables stable suspension standing culture (e.g., not more than 0.025% (weight/volume), not more than 0.02% (weight/volume)). When adherent cells are subjected to suspension culture while being attached to a nanofiber composed of water-insoluble polysaccharides, the cells can be maintained and proliferated at a higher density than when they are adhered to the bottom surface of a culture container and single layer cultured.

When adherent cells are subjected to suspension culture while being attached to a nanofiber composed of water-insoluble polysaccharides to maintain or proliferate the adherent cells, a medium permitting maintenance or proliferation of the adherent cells while maintaining the character thereof is used as the medium contained in the medium composition of the present invention to be used for the suspension culture. Those of ordinary skill in the art can appropriately select the medium according to the kind of the adherent cells.

In one embodiment, mammalian stem cells (e.g., mesenchymal stem cell) or progenitor cells (e.g., pre-adipocyte) are maintained or proliferated by performing suspension culture of the cells while they are attached to a chitin nanofiber. By the suspension culture, mammalian stem cells (e.g., mesenchymal stem cell) or progenitor cells (e.g., pre-adipocyte) can be maintained or proliferated while maintaining the character (e.g., differentiation potency) thereof.

When adherent cells are subjected to suspension culture while being attached to a nanofiber composed of water-insoluble polysaccharides, the adherent cells can be passaged by simply adding a fresh medium or the medium composition of the present invention to the suspension culture, or just adding a culture after culture entirely or partly to a fresh medium or the medium composition of the present invention, without a detaching operation of the cells from a culture container. Therefore, using the passage culture method of the present invention, adherent cells can be passaged without a detaching operation of the cells from a culture container. Using this passage culture method, moreover, the culture scale of adherent cells can be expanded without a detaching operation of the cells from a culture container. As a detaching operation of the cells from a culture container, a treatment with a chelating agent (e.g., EDTA) and/or protease (e.g., trypsin, collagenase) can be mentioned. The above-mentioned passage culture method is advantageous for passage culture of adherent cells highly sensitive to a detaching operation of the cells from a culture container (e.g., adherent cell with viability decreased by detaching operation, adherent cell with character susceptible to change by detaching operation). Examples of the adherent cells highly sensitive to a detaching operation of the cells from a culture container include, but are not limited to, stem cell (e.g., mesenchymal stem cell), progenitor cell (e.g., pre-adipocyte), primary cultured cells and the like.

### (1-2) Differentiation induction of adherent cells

When suspension culture of adherent cells is performed while the cells are attached to a nanofiber composed of water-insoluble polysaccharides, differentiation of the adherent cell into a desired cell can be induced by performing the suspension culture under appropriate differentiation induction conditions. As mentioned above, when suspension culture of adherent cells is performed while the cells are attached to a nanofiber composed of water-insoluble polysaccharides, the proliferation thereof is promoted. Thus, good differentiation induction of the adherent cell into a desired cell can be expected by performing the suspension culture under appropriate differentiation induction conditions.

The differentiation induction conditions can be appropriately determined according to the kind of the adherent cell and the desired differentiated cell. Those of ordinary skill in the art can differentiate a particular adherent cell into a desired cell by applying various known differentiation induction conditions to the above-mentioned suspension culture. For example, a particular differentiation-inducing factor is added to the above-mentioned medium composition of the present invention and suspension culture of adherent cells is performed in the presence of the differentiation-inducing factor, whereby differentiation into a desired cell can be induced.

The differentiation induction conditions applicable in the present invention are not particularly limited. For example, the following can be mentioned.
- differentiation of mesenchymal stem cell into adipocyte: Using isobutylmethylxanthine (IBMX), dexamethasone, insulin and indomethacin as adipocyte differentiation-inducing factors, mesenchymal stem cells are cultivated in the presence of these factors.
- differentiation of mesenchymal stem cell into osteocyte: Using dexamethasone, ascorbic acid and β glycerophosphate as osteocyte differentiation-inducing factors, mesenchymal stem cells are cultivated in the presence of these factors.
- differentiation of mesenchymal stem cell into chondrocyte: Using insulin, TGF-β3 and ascorbic acid as chondrocyte differentiation-inducing factors, mesenchymal stem cells are cultivated in the presence of these factors.

The conditions of adipocyte differentiation, osteocyte differentiation or chondrocyte differentiation of mesenchymal stem cells are described in, for example, the following papers.
[1] da Silva Meirelles L, Caplan AI, NardiNB., Stem Cells 2008; 26(9):2287-99.
[2] Crisan M, Yap S, CasteillaL, et al., Cell Stem Cell 2008; (3):301-13.
[3] Dominici M, Le Blanc K, Mueller I, Slaper-Cortenbach I, et al., Cytother2006; 8(4):315-7.
[4] Caplan AI., Cell Stem Cell 2008; 3(3):229-30.
   - differentiation of pre-adipocyte into adipocyte: Using isobutylmethylxanthine (IBMX), dexamethasone, insulin and indomethacin as adipocyte differentiation-inducing factors, pre-adipocytes are cultivated in the presence of these factors.

   The conditions of adipocyte differentiation of pre-adipocytes are described in, for example, the following papers.
[5] Hutley L J, Newell F M, et al., Eur J Clin Invest. 2003; 33(7): 574-81.
[6] Yin Y, Yuan H, et al., Mol Endocrinol. 2006; 20(2):268-78.
[7] Rival Y, Stennevin A, et al., J Pharmacol Exp Ther. 2004; 311(2): 467-75.
   In addition, the following differentiation induction conditions are known and applicable to the present invention. . differentiation of pre-adipocyte into osteocyte:
[8] Shirakawa K, Maeda S, et al., Mol Cell Biol. 2006; 26(16):6105-16.

The suspension culture of adherent cells under differentiation induction conditions is performed until desired differentiated cells emerge. Emergence of the desired differentiated cells can be confirmed by examining expression of the differentiation marker of the cells and the like. As the result of the suspension culture, the desired differentiated cells can be obtained while they are attached to a nanofiber composed of water-insoluble polysaccharides.

In one embodiment, mammalian mesenchymal stem cells attached to a chitosan nanofiber are subjected to suspension culture under differentiation induction conditions into adipocyte, osteocyte or chondrocyte. Suspension culture is performed until adipocyte, osteocyte or chondrocyte emerges, whereby adipocyte, osteocyte or chondrocyte is obtained. The adipocyte, osteocyte or chondrocyte may be obtained while being attached to a chitosan nanofiber.

In one embodiment, mammalian pre-adipocytes attached to a chitosan nanofiber are subjected to suspension culture under differentiation induction conditions into adipocyte. Suspension culture is performed until adipocyte emerges, whereby adipocyte is obtained. The adipocyte may be obtained while being attached to a chitosan nanofiber.

### (1-3) Production of humoral factor by adherent cells

When suspension culture of adherent cells is performed while the cells are attached to a nanofiber composed of water-insoluble polysaccharides, the adherent cells can be cultured at a high density and efficiently proliferated. Thus, the suspension culture is useful for production of a humoral factor by in vitro cell culture. The humoral factor can be obtained by subjecting the adherent cells that produce a desired humoral factor to suspension culture while the cells are attached to a nanofiber composed of water-insoluble polysaccharides and isolating the intended humoral factor from the culture (e.g., culture supernatant). Examples of the humoral factor include, but are not limited to, antibody, enzyme (urokinase etc.), hormone (insulin etc.), cytokine (interferon, interleukin, tumor necrosis factor, colony stimulating factor, growth factor etc.), vaccine antigen, and other physiologically active substances (protein, peptide etc.). The cells that produce humoral factor includes untransformed cells such as skin cell, chondrocyte, hepatocyte, pancreatic cell, kidney cell, mesenchymal stem cell, adipocyte and the like, and transformed cells into which a gene encoding a humoral factor, or a gene involved in the biosynthesis of useful substances has been introduced. The cell that produces desired humoral factor preferably secretes a humoral factor extracellularly. Specific examples of the cell that produces humoral factor include, but are not limited to, HEK293, CHO-K1, BHK-21, MDCK, Vero, HepG2, MCF-7 and the like into which a gene encoding a humoral factor or a gene involved in the biosynthesis of a humoral factor has been introduced. The cells used for the production of a humoral factor such as recombinant protein and the like is well known to those of ordinary skill in the art, and such cells can be used for the method of the present invention. The culture scale may be expanded by a maintenance or proliferation method of adherent cells described in the above-mentioned (1-1). To isolate a humoral factor from a culture, cells need to be removed from the culture. When the above-mentioned suspension culture is used, since the adherent cells are suspended in the medium composition while being attached to a nanofiber composed of water-insoluble polysaccharides, the cells can be removed by a convenient method such as centrifugation, filtration treatment and the like. In addition, nanofiber in the medium composition can also be removed by a convenient method such as centrifugation, filtration treatment and the like. A method for isolating a humoral factor from a culture is well known to those of ordinary skill in the art and, for example, a biochemical separation and purification method of bioactive substances such as chromatography (e.g., chromatographys such as ion exchange chromatography, hydrophobic chromatography, affinity chromatography, reversed-phase chromatography and the like) and the like can be applied.

In one embodiment, mammalian mesenchymal stem cell, pre-adipocyte or adipocyte is subjected to suspension culture while being attached to a chitosan nanofiber, and a useful substance such as cytokine, exosome and the like extracellularly secreted by these cells is isolated from the obtained culture supernatant. The adipocyte may be induced from mammalian mesenchymal stem cells or pre-adipocytes by the method of the above-mentioned (1-2).

### (2) Preservation and/or transportation under non-freezing conditions

Adherent cells show high survivability while being adhered to nanofibers composed of water-insoluble polysaccharides. In addition, adherent cells can be concentrated at a high density without separation from a scaffold. Therefore, nanofibers composed of water-insoluble polysaccharides can be used as a carrier for preservation and/or transportation of adherent cells under non-freezing conditions. Preservation and/or transportation of adherent cells under non-freezing conditions using the nanofiber can be performed by preserving and/or transporting the adherent cells using the above-mentioned medium composition of the present invention while the cells are attached to the nanofiber composed of water-insoluble polysaccharides and suspended in the medium composition (preferably, under suspension standing condition).

The medium composition of the present invention to be used for preservation and/or transport may contain, in addition to the aforementioned composition, various components having a cell life-prolonging effect, during preservation of cells and tissues in a non-frozen state. Examples of the component include saccharides (excluding polysaccharides) (e.g., monosaccharides, disaccharides), antioxidants (e.g., SOD, vitamin E or glutathione), hydrophilic polymers (e.g., polyvinylpyrrolidone), chelating agents (e.g., EDTA), sugar alcohols (e.g., mannitol, sorbitol), glycerol and the like.

For preservation and/or transportation, for example, the desired adherent cells are dispersed in the medium composition of the present invention to allow the adherent cells to attach to a nanofiber composed of water-insoluble polysaccharides, whereby a suspension of the adherent cells attached to the nanofibers composed of water-insoluble polysaccharides is obtained. The culture containing the adherent cells attached to the nanofibers composed of water-insoluble polysaccharides, which is obtained by the suspension culture in the above-mentioned (1) may be directly subjected to preservation and/or transportation. It is also possible to obtain a suspension of the adherent cells attached to the nanofibers composed of water-insoluble polysaccharides by subjecting the culture obtained by the suspension culture in the above-mentioned (1) to centrifugation and the like to recover the adherent cells attached to the nanofiber composed of water-insoluble polysaccharides, and suspending same in an appropriate liquid medium. The concentration of the adherent cells in the suspension is not particularly limited and is generally 1×10² - 5×10⁶ cells/mL, preferably 1×10⁴ - 1×10⁶ cells/mL.

For preservation and/or transportation, the above-mentioned suspension of the adherent cells is placed in a tight-sealable container. Examples of the container include, but are not limited to, flask, plastic bag, Teflon (registered trade mark) bag, tube, culture bag and the like. To avoid leakage of the contents and contamination with bacterium and the like from the outside world during preservation or transport, a container containing a suspension of the adherent cells is preferably tightly sealed.

While the temperature during preservation and/or transport is not particularly limited as long as the survival of the adherent cells or tissue is maintained, it is generally not more than 37°C. A decrease in the viability of cell or tissue during preservation and/or transport can be avoided when the temperature is low. However, to prevent freezing of cells or tissues, they are generally preserved and/or transported at a temperature exceeding the melting point of the medium composition of the present invention. Therefore, the temperature during preservation and/or transport is generally maintained at -5 - 42°C, preferably 1 - 37°C, more preferably 4 - 32°C, further preferably 18 - 30°C.

The period of preservation and/or transport is not particularly limited as long as the desired adherent cells can be maintained in a viable state in the medium composition of the present invention. It is generally not less than 1 hr, within 10 days, preferably 1 - 8 days, more preferably 1 - 3 days. During the preservation and/or transport period, the adherent cells are preferably maintained in a suspension stand state in the medium composition of the present invention while being attached to a nanofiber composed of water-insoluble polysaccharides.

Using the preservation or transportation method of the present invention, adherent cells can be preserved and/or transported under non-freezing conditions without separating from the scaffold while maintaining the suspension state. Therefore, adherent cells can be preserved and/or transported while minimizing damage due to freezing of the cells, detachment of adherent cells from a scaffold, coagulation of cells contacted by sediment formation and the like, and maintaining the inherent functions.

In one embodiment, mesenchymal stem cell, pre-adipocyte, adipocyte, osteocyte or chondrocyte of a mammal is preserved and/or transported under non-freezing condition while being attached to a chitosan nanofiber. The adipocyte, osteocyte or chondrocyte may be induced from mesenchymal stem cell or pre-adipocyte of a mammal by the method of the above-mentioned (1-2) .

When preserved and/or transported adherent cells are recovered, the adherent cells may be detached from a nanofiber by treating the adherent cells with a chelating agent (e.g., EDTA) and/or protease (e.g., trypsin, collagenase), or the adherent cells may be detached from a nanofiber by degrading the nanofiber with an appropriate degrading enzyme by the below-mentioned recovery method of the present invention. The adherent cells preserved and/or transported while being attached to a nanofiber may be suspension cultured by the method described in the above-mentioned (1-1) and then a recovery operation may be performed. Alternatively, the adherent cells attached to a nanofiber may be transferred to a cell adhesive incubator and successively cultured. The cell adhesive incubator may be coated with any cell supporting substrate such as an extracellular matrix (ECM) and the like for the purpose of improving adhesion with cells on the surface of the incubator. Examples of the cell supporting substrate include, but are not limited to, collagen, gelatin, poly-L-lysine, poly-D-lysine, laminin, and fibronectin, a mixture thereof, for example, Matrigel and the like. When the adherent cells attached to a nanofiber are cultured in a cell adhesive incubator, the adherent cells move from the nanofiber to the surface of a cell adhesive incubator and proliferate while being adhered to the surface of the incubator. The cells adhered to the surface of the incubator may be recovered by detaching from the surface by a treatment with a chelating agent (e.g., EDTA) and/or protease (e.g., trypsin, collagenase).

### (3) transplantation

As mentioned above, adherent cells show high survivability while being adhered to nanofibers composed of water-insoluble polysaccharides. In addition, adherent cells can be concentrated at a high density while maintaining good functions and without separation from a scaffold. Since water-insoluble polysaccharides such as chitin and the like are biodegradable, they are degraded and disappear after transplantation to the body. Therefore, nanofibers composed of water-insoluble polysaccharides can be used as a carrier for transplantation of adherent cells to a living body. Transplantation of an effective amount of the adherent cells attached to a nanofiber composed of water-insoluble polysaccharides to patients in need of a treatment with the adherent cells can treat diseases and disorders in the patients. The diseases and disorders may be caused by deletion, shortage or functional disorder of the adherent cells. For example, transplantation of an effective amount of human adherent cells attached to a nanofiber composed of water-insoluble polysaccharides to human patients in need of a treatment with the adherent cells can treat diseases and disorders in the human patients. The adherent cells to be transplanted may be obtained by the suspension culture in the above-mentioned (1), or the preservation and/or transportation under non-freezing conditions in the above-mentioned (2).

In one embodiment, transplantation of mesenchymal stem cell, pre-adipocyte, adipocyte, osteocyte or chondrocyte of a mammal attached to a chitosan nanofiber to patients in need of a treatment with the mesenchymal stem cell, pre-adipocyte, adipocyte, osteocyte or chondrocyte can treat diseases and disorders in the patients. The diseases and disorders may be caused by deletion, shortage or functional disorder of the mesenchymal stem cell, pre-adipocyte, adipocyte, osteocyte or chondrocyte. The mammal is preferably a human. The mesenchymal stem cell or pre-adipocyte to be transplanted may be maintained or proliferated by the method of the above-mentioned (1-1). The adipocyte, osteocyte or chondrocyte may be induced from mesenchymal stem cell or pre-adipocyte of a mammal by the method of the above-mentioned (1-2).

### [Continuous treatment]

As mentioned above, nanofibers composed of water-insoluble polysaccharides can be used as common carriers for various operations such as (1) suspension culture (maintenance or proliferation, differentiation induction or production of humoral factor), (2) preservation and/or transportation under non-freezing conditions, and (3) transplantation and the like of adherent cells such as mesenchymal stem cell, pre-adipocyte and the like. Thus, using nanofibers composed of water-insoluble polysaccharides, plural treatments selected from (1) suspension culture ((1-1) maintenance or proliferation, (1-2) differentiation induction, or (1-3) humoral factor production), (2) preservation and/or transportation under non-freezing conditions, and (3) transplantation can be performed continuously. The "continuously" means subjecting adherent cells attached to a nanofiber composed of water-insoluble polysaccharides obtained by a particular operation to a next operation while maintaining the attached state. The survivability and function of adherent cells can be maintained in a good state by continuously performing plural operations without detaching the adherent cells from a scaffold.

The combination of treatments and the order thereof are not particularly limited and can be appropriately determined according to the object. The same treatment may be performed plural times continuously or non-continuously. Examples of the combination of treatments and the order thereof include, but are not limited to, the following.
(a) (1-1)→H2)
(b) (1-1)→(2)→(1-1)
(c) (1-1)→(2)→(1-2)
(d) (1-1)→(2)→(1-1)→(1-2)
(e) (1-1)→(2)→(1-2)→(1-1)
(f) (1-1)→(2)→(1-1)→(1-2)→(1-1)
(g) (1-1)→(1-2)→(2)
(h) (1-1)→(1-2)→(1-1)→(2)
(i) (1-1)→(1-2)→(2)→(1-1)
(j) (1-1)→(1-2)→(1-1)→(2)→(1-1)
(k) (1-1)→(1-2)
(l) (1-1)→(1-2)→(1-1)
(m) after operation of any of the aforementioned (a) - (l) (e.g., (a), (b), (c) or (k)), continuously (3)
(n) after operation of any of the aforementioned (a) - (l) (e.g., (a), (b), (c) or (k)), continuously (1-3)

In the above-mentioned combinations, the nanofiber composed of water-insoluble polysaccharides is preferably a chitin nanofiber. In operation (1-1) in the above-mentioned (a) and (b), the adherent cell to be maintained or proliferated is preferably a mammalian mesenchymal stem cell, pre-adipocyte, adipocyte, osteocyte or chondrocyte. The adherent cell to be maintained or proliferated in operation (1-1) to be performed before operation (1-2) in the above-mentioned (c), (d), (e), (f), (g), (h), (i), (j), (k) and (l) is preferably a mammalian mesenchymal stem cell or pre-adipocyte. The adherent cell to be maintained or proliferated in operation (1-1) to be performed after operation (1-2) in the above-mentioned (e), (f), (h), (i), (j) and (1) is preferably an adipocyte, osteocyte or chondrocyte. In the above-mentioned combinations, operation (1-2) is preferably differentiation induction of a mammalian mesenchymal stem cell or pre-adipocyte into an adipocyte, osteocyte or chondrocyte.

### [Recovery of adherent cells attached to nanofiber]

The present invention provides a method for recovering adherent cells from adherent cells attached to a nanofiber composed of water-insoluble polysaccharides, including treating the adherent cells attached to the nanofiber composed of water-insoluble polysaccharides with a degrading enzyme of the water-insoluble polysaccharides to degrade the nanofiber, detaching the adherent cells from the nanofiber and recovering the adherent cells.

Generally, when adherent cells attached to a culture container and the like are recovered by detaching, a treatment with a chelating agent (e.g., EDTA) and/or protease (e.g., trypsin, collagenase) is performed. When this treatment is performed, the structure of an extracellular matrix of the adherent cells may be destroyed, or the adherent cells may be damaged and the survivability and function thereof may be impaired by a direct action of a chelating agent or protease on the adherent cells. When the cells are recovered using a chelating agent or protease, it is necessary to remove the medium and wash the cells with PBS and the like to remove divalent ion, protein and the like contained in the medium, and this washing operation may damage the cells. Particularly, stem cells (e.g., mesenchymal stem cell), progenitor cells (e.g., pre-adipocyte), primary cultured cells and the like have high sensitivity to a detaching operation using the aforementioned chelating agent and protease. In contrast, in the recovery method of the present invention, water-insoluble polysaccharides constituting the nanofiber to which the adherent cells are attached are degraded. Therefore, destruction of the structure of the extracellular matrix of the adherent cells can be minimized. Since mammalian cells and the like generally do not contain water-insoluble polysaccharides as a constituent component thereof, even when the cells are treated with a degrading enzyme of water-insoluble polysaccharides, the direct influence on the cells can be minimized. Furthermore, since mammalian medium and the like generally do not contain water-insoluble polysaccharides, a washing operation with PBS and the like is not necessary. Therefore, according to the recovery method of the present invention, adherent cells can be recovered by detaching from the nanofiber while minimizing the damage on the adherent cells.

In the recovery method of the present invention, a degrading enzyme of the water-insoluble polysaccharides is appropriately selected according to the kind thereof. For example, when chitin or chitosan is used as the water-insoluble polysaccharide, chitinase, chitobiase, chitosanase, β-1,3-glucanase and the like can be used as the degrading enzyme thereof. When cellulose is used as the water-insoluble polysaccharide, cellulase (endoglucanase (EC 3.2.1.4) ("EG"), exoglucanase or cellobiohydrolase (EC 3.2.1.91) ("CBH"), β-glucosidase ([β]-D-glucosideglucohydrolase; EC 3.2.1.21) ("BG")) can be used as the degrading enzyme thereof. When a cellulose material contains a large amount of hemicellulose, xylanase or mannanase is preferably added besides cellulose as an enzyme for degrading hemicellulose. Plural degrading enzymes may be used in combination. For example, when chitin or chitosan is used as the water-insoluble polysaccharide, a mixture of 2, 3 or 4 enzymes selected from the group consisting of chitinase, chitobiase, chitosanase and β-1,3-glucanase may also be used as the degrading enzyme thereof. Yatalase (Takara Bio Inc.) is a mixture of chitinase, chitobiase, chitosanase and β-1,3-glucanase, and can be preferably used as a degrading enzyme of chitin or chitosan in the recovery method of the present invention.

For example, a degrading enzyme of the water-insoluble polysaccharides is added to a suspension of adherent cells attached to nanofibers composed of water-insoluble polysaccharides and the mixture is incubated for a time sufficient for the detachment of the adherent cells. As mentioned above, since the detachment does not require a chelating agent (EDTA) or a protease (trypsin, collagenase), in one embodiment, a degrading enzyme of the water-insoluble polysaccharides is added to the suspension without adding a chelating agent (EDTA) and/or protease (e.g., trypsin, collagenase). In addition, since a washing operation with PBS and the like is not necessary, in one embodiment, a degrading enzyme of the water-insoluble polysaccharides is added to the medium without performing a washing operation to remove divalent ion and/or protein in the medium in which the adherent cells are suspended (e.g., setting the concentration to not more than 1/10). The temperature of incubation by the degrading enzyme of the water-insoluble polysaccharides is generally 20°C - 37°C. The incubation time varies depending on the kind of the enzyme and the like and is generally 5 - 60 min.

When the nanofiber is degraded and the adherent cells are detached from the nanofiber, the detached adherent cells can be recovered by subjecting the suspension to centrifugation.

Since the damage on the thus-recovered adherent cells is minimized, the cells can be preferably used for functional analysis, transplantation and the like.

For example, in the treatment of adherent cells using the aforementioned nanofiber composed of water-insoluble polysaccharides, the adherent cells may be detached and recovered from the nanofiber by the recovery method of the present invention after operations of (1) suspension culture ((1-1) maintenance or proliferation, (1-2) differentiation induction) or (2) preservation and/or transportation under non-freezing conditions. The adherent cells may be detached and recovered from the nanofiber by the recovery method of the present invention after the above-mentioned continuous operations (e.g., (a) - (l)).

The present invention is explained in more detail in the following by specifically describing the Examples of the medium composition of the present invention. However, the present invention is not limited thereby.

### [Examples]

### (Experimental Example 1: Proliferation of human pre-adipocyte by 3D culture using chitin nanofiber)

Chitin nanofiber (biomass nanofiber BiNFi-S 2 mass %, Sugino Machine Limited, Lot. GG30-G30) and deacylated gellan gum (DAG) (KELCOGEL CG-LA, manufactured by SANSHO Co., Ltd.) were each suspended in ultrapure water (Milli-Q water) to 1% (w/v) and dispersed by stirring with heating at 90°C, and the aqueous solution was autoclave sterilized at 121°C for 20 min. A medium composition obtained by adding a chitin nanofiber to a human pre-adipocyte proliferation medium (#CAS11K500, manufactured by TOYOBO CO., LTD.) (final concentration: 0.003% (w/v), 0.01% (w/v), 0.03% (w/v)), a medium composition added with deacylated gellan gum (final concentration: 0.015% (w/v), 0.03% (w/v)), and a no-addition medium composition free of the above-mentioned substrate were prepared. Successively, the cultured human pre-adipocytes (derived from subcutaneous tissue, #CAS02s05a, manufactured by TOYOBO CO., LTD.) were suspended in each of the above-mentioned medium compositions at 33333 cells/mL, and seeded in a 96 well flat bottom ultra low attachment surface microplate (manufactured by Corning Incorporated, #3474) at 150 µL/well. The cells were cultured in a CO₂ incubator (37°C, 5% CO₂) in a static state for 10 days. An ATP reagent (150 µL, CellTiter-Glo™ Luminescent Cell Viability Assay, manufactured by Promega) was added to the culture mediums on days 3, 7, 10, and the cells were suspended and stood for about 10 min at room temperature. The luminescence intensity (RLU value) was measured by FlexStation3 (manufactured by Molecular Devices) and the luminescence value of the medium alone was subtracted to measure the number of viable cells as a mean of 4 samples.

As a result, when human pre-adipocytes are cultured using a medium composition added with a chitin nanofiber, a cell proliferation action was found at a concentration of not less than 0.003% (w/v). The RLU value (ATP measurement, luminescence intensity) in each culture is shown in Table 1.

**[Table 1]**

| | Day 0 | Day 3 | Day 7 | Day 10 |
|---|---|---|---|---|
| not added | 13140 | 13061 | 4673 | 2395 |
| DAG | 13722 | 14974 | 8762 | 7560 |
| 0.015% | | | | |
| DAG | 13620 | 14468 | 8221 | 7125 |
| 0.003% | | | | |
| chitin nanofiber | 14284 | 18541 | 20253 | 20114 |
| 0.003% | | | | |
| chitin nanofiber | 13760 | 18914 | 19413 | 21967 |
| 0.01% | | | | |
| chitin nanofiber | 13538 | 18517 | 19550 | 22909 |
| 0.03% | | | | |

### (Experimental Example 2: Proliferation of human adipose tissue-derived mesenchymal stem cells by 3D culture using chitin nanofiber)

Chitin nanofiber (biomass nanofiber BiNFi-S 2 mass %, Sugino Machine Limited, Lot. GG30-G30) was suspended in ultrapure water (Milli-Q water) to 1% (w/v), dispersed by stirring with heating at 90°C, and the aqueous solution was autoclave sterilized at 121°C for 20 min. A medium composition obtained by adding a chitin nanofiber to a mesenchymal stem cell proliferation medium (C-28009, manufactured by Takara Bio Inc.) which is a low serum medium (final concentration: 0.001% (w/v), 0.003% (w/v), 0.01% (w/v), 0.03% (w/v)), and a no-addition medium composition free of the above-mentioned substrate were prepared. Successively, the cultured human adipose-derived mesenchymal stem cells (C-12977, manufactured by Takara Bio Inc.) were suspended in each of the above-mentioned medium compositions at 13333 cells/mL, and seeded in a 96 well flat bottom ultra low attachment surface microplate (manufactured by Corning Incorporated, #3474) at 150 µL/well. The cells were cultured in a CO₂ incubator (37°C, 5% CO₂) in a static state for 10 days. An ATP reagent (150 µL, CellTiter-Glo™ Luminescent Cell Viability Assay, manufactured by Promega) was added to the culture mediums on days 3, 7, 10, and the cells were suspended and stood for about 10 min at room temperature. The luminescence intensity (RLU value) was measured by FlexStation3 (manufactured by Molecular Devices) and the luminescence value of the medium alone was subtracted to measure the number of viable cells as a mean of 4 samples.

As a result, when human adipose tissue-derived mesenchymal stem cells were cultured using a medium composition added with a chitin nanofiber, a cell proliferation promoting action was found at a concentration of not less than 0.001% (w/v). The RLU value (ATP measurement, luminescence intensity) in each culture is shown in Table 2.

**[Table 2]**

| | Day 0 | Day 3 | Day 7 | Day 10 |
|---|---|---|---|---|
| not added | 1562 | 2646 | 3049 | 3954 |
| chitin nanofiber | 1488 | 3278 | 5721 | 6276 |
| 0.001% | | | | |
| chitin nanofiber | 1455 | 3071 | 3759 | 4868 |
| 0.003% | | | | |
| chitin nanofiber | 1646 | 5150 | 15716 | 15162 |
| 0.01% | | | | |
| chitin nanofiber | 1744 | 5106 | 15434 | 15386 |
| 0.03% | | | | |

### (Experimental Example 3: Recovery of human pre-adipocytes adhered to chitin nanofiber)

Chitin nanofiber (biomass nanofiber BiNFi-S 2 mass %, Sugino Machine Limited, Lot. GG30-G30) was suspended in ultrapure water (Milli-Q water) to 1% (w/v), dispersed by stirring with heating at 90°C, and the aqueous solution was autoclave sterilized at 121°C for 20 min. A medium composition obtained by adding a chitin nanofiber to a human pre-adipocyte proliferation medium (#CAS11K500, manufactured by TOYOBO CO., LTD.) (final concentration: 0.03% (w/v)) was prepared. Successively, the cultured human pre-adipocytes (derived from subcutaneous tissue, #CAS02s05a, manufactured by TOYOBO CO., LTD.) were suspended in the above-mentioned medium composition at 33333 cells/mL, and seeded in a 96 well flat bottom ultra low attachment surface microplate (manufactured by Corning Incorporated, #3474) at 150 µL/well. The cells were cultured in a CO₂ incubator (37°C, 5% CO₂) in a static state for 3 days, and the cells were adhered to the chitin nanofiber.

After 3 days of culture, Yatalase (manufactured by Takara Bio Inc., T017) containing a chitin degrading enzyme was added to each well at a final concentration of 0.1% (w/v) or 0.25% (w/v) and the mixture was stood at 37°C for about 1 hr. Thereafter, 4 wells of cell suspension containing chitin nanofiber degradation products and human pre-adipocytes were collected in a 15 mL tube and centrifuged at 1500 rpm. After centrifugation, the medium components in the supernatant were removed, precipitated human pre-adipocytes were suspended in 600 µL of a human pre-adipocyte proliferation medium (#CAS11K500, manufactured by TOYOBO CO., LTD.), re-seeded in a 96 well flat bottom microplate (manufactured by Corning Incorporated, #3585) at 150 µL/well and photographed.

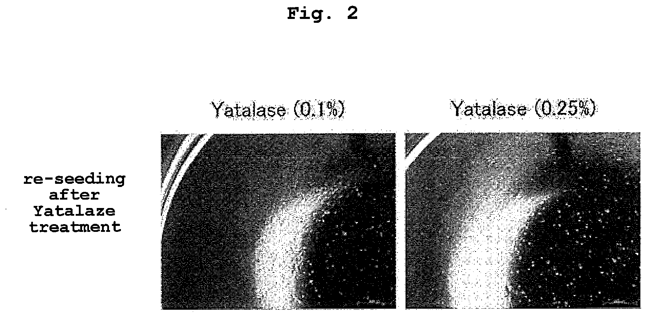

As a result, when human pre-adipocytes are cultured in the medium composition added with the chitin nanofiber, human pre-adipocytes adhered to the chitin nanofiber and it was found that human pre-adipocytes adhered to the chitin nanofiber could be isolated in a single cell-like form when the chitin nanofiber was digested using Yatalase. A photograph of the cells after Yatalase treatment is shown in Fig. 2.

### (Experimental Example 4: Recovery and re-seeding of human pre-adipocytes adhered to chitin nanofiber)

Chitin nanofiber (biomass nanofiber BiNFi-S 2 mass %, Sugino Machine Limited, Lot. GG30-G30) was suspended in ultrapure water (Milli-Q water) to 1% (w/v), dispersed by stirring with heating at 90°C, and the aqueous solution was autoclave sterilized at 121°C for 20 min. A medium composition obtained by adding a chitin nanofiber to a human pre-adipocyte proliferation medium (#CAS11K500, manufactured by TOYOBO CO., LTD.) (final concentration: 0.03% (w/v)) was prepared. Successively, the cultured human pre-adipocytes (derived from subcutaneous tissue, #CAS02s05a, manufactured by TOYOBO CO., LTD.) were suspended in the above-mentioned medium composition at 33333 cells/mL, and seeded in a 96 well flat bottom ultra low attachment surface microplate (manufactured by Corning Incorporated, #3474) at 150 µL/well. The cells were cultured in a CO₂ incubator (37°C, 5% CO₂) in a static state for 3 days, and the cells were adhered to the chitin nanofiber.

After 3 days of culture, Yatalase (manufactured by Takara Bio Inc., T017) containing a chitin degrading enzyme was added to each well at a final concentration of 0.1% (w/v) or 0.25% (w/v) and the mixture was stood at 37°C for about 1 hr. Thereafter, 4 wells of cell suspension containing chitin nanofiber degradation products and human pre-adipocytes were collected in a 15 mL tube and centrifuged at 1500 rpm. After centrifugation, the medium components in the supernatant were removed, precipitated human pre-adipocytes were suspended in 600 µL of a human pre-adipocyte proliferation medium (#CAS11K500, manufactured by TOYOBO CO., LTD.), re-seeded in a 96 well flat bottom microplate (manufactured by Corning Incorporated, #3585) at 150 µL/well and the levels of cell proliferation and differentiation induction into adipocyte were evaluated.

The cell proliferation was evaluated using the measurement method of ATP value. After re-seeding, the cells were cultured in a CO₂ incubator (37°C, 5% CO₂) in a static state for 7 days at maximum. An ATP reagent (150 µL, CellTiter-Glo™ Luminescent Cell Viability Assay, manufactured by Promega) was added to the culture medium on days 0, 3, 7 after re-seeding and the mixture was stood for about 10 min at room temperature. The luminescence intensity (RLU value) was measured by FlexStation3 (manufactured by Molecular Devices) and the luminescence value of the medium alone was subtracted to calculate the number (mean of 4 samples) of viable cells.

The differentiation into adipocyte was evaluated using mRNA expression value as an adipocyte marker. The cells were re-seeded and cultured in a static state in a CO₂ incubator (37°C, 5% CO₂) for 3 days. Adhesion of the cells to a 96 well flat bottom microplate was confirmed, the medium was removed and replaced with a human adipocyte differentiation medium (#CAS11D250, manufactured by TOYOBO CO., LTD.) at 150 µL/well. The cells were cultured in a CO₂ incubator (37°C, 5% CO₂) in a static state for 10 days.

Total RNA was extracted from the adhered cells using RNeasy Mini kit (manufactured by QIAGEN) on replacement with the differentiation medium (day 0) and day 10. Using PrimeScript™ RT Master Mix (manufactured by Takara Bio Inc.), a reverse transcription reaction was performed on GeneAmp PCR System 9700 (manufactured by Applied Biosystems) to synthesize cDNA from total RNA. Each of the obtained cDNA samples diluted 1/10 with sterilized water was used as a PCR template. In addition, the dispensed and mixed cDNA was used as a sample for preparing an analytical curve. The analytical curve was set in a quantitative range from 1/3 to 1/243 dilution at a 3-fold common ratio. PCR was performed using each cDNA sample, analytical samples, Premix Ex Taq™ (manufactured by Takara Bio Inc.) and various TaqMan probes (manufactured by Applied Biosystems) and 7500 Real Time PCR System (manufactured by Applied Biosystems). PPIA (cyclophilin B) mRNA was used as an endogenous control, and the expression of PPAR gamma mRNA or lipoprotein lipase (LPL) mRNA was corrected with the value of PPIA. The probes used (manufactured by Applied Biosystems) are shown in the following.
PPIA: HS99999904
PPAR gamma: HS011155134
LPL: HS00173425

As a result, when human pre-adipocytes were cultured in the medium composition added with the chitin nanofiber, human pre-adipocytes adhered to the chitin nanofiber and it was found that human pre-adipocytes adhered to the chitin nanofiber could be isolated in a single cell-like form when the chitin nanofiber was digested using Yatalase. In addition, the isolated cells maintained proliferation potency and differentiation potency into adipocyte on a 96 well flat bottom microplate. The RLU value (ATP measurement, luminescence intensity) relating to cell proliferation evaluation is shown in Table 3 and PPAR gamma and LPL mRNA expression value relating to adipocyte differentiation evaluation are respectively shown in Table 4 and Table 5.

**[Table 3]**

| | Day 0 | Day 3 | Day 7 |
|---|---|---|---|
| Yatalase | 9304 | 21547 | 57172 |
| 0.1 % | | | |
| Yatalase | 15017 | 16919 | 53602 |
| 0.25 % | | | |

**[Table 4]**

| PPAR gamma | Day 0 | Day 10 |
|---|---|---|
| (PPIA corrected value) | | |
| Yatalase | 0.12 | 1.28 |
| 0.1 % | | |
| Yatalase | 0.10 | 1.08 |
| 0.25 % | | |

**[Table 5]**

| LPL gamma | Day 0 | Day 10 |
|---|---|---|
| (PPIA corrected value) | | |
| Yatalase | 0 | 1.40 |
| 0.1 % | | |
| Yatalase | 0 | 1.28 |
| 0.25 % | | |

### (Experimental Example 5: Differentiation induction of human pre-adipocytes adhered to chitin nanofiber)

Chitin nanofiber (biomass nanofiber BiNFi-S 2 mass %, Sugino Machine Limited, Lot. GG30-G30) was suspended in ultrapure water (Milli-Q water) to 1% (w/v), dispersed by stirring with heating at 90°C, and the aqueous solution was autoclave sterilized at 121°C for 20 min. Using this solution, a medium composition obtained by adding a chitin nanofiber to a human adipocyte differentiation medium (#CAS11D250, manufactured by TOYOBO CO., LTD.) (final concentration: 0.03% (w/v)) was prepared. Successively, the cultured human pre-adipocytes (derived from subcutaneous tissue, #CAS02s05a, manufactured by TOYOBO CO., LTD.) were suspended in the above-mentioned medium composition at 33333 cells/mL, and seeded in a 96 well flat bottom ultra low attachment surface microplate (manufactured by Corning Incorporated, #3474) at 150 µL/well. The cells were cultured in a CO₂ incubator (37°C, 5% CO₂) in a static state for 14 days at maximum.

The cell survivability was evaluated using the measurement method of ATP value. An ATP reagent (150 µL, CellTiter-Glo™ Luminescent Cell Viability Assay, manufactured by Promega) was added to the culture medium on days 0, 4, 8, 14 and the mixture was stood for about 10 min at room temperature. The luminescence intensity (RLU value) was measured by FlexStation3 (manufactured by Molecular Devices) and the luminescence value of the medium alone was subtracted to calculate the number (mean of 4 samples) of viable cells.

The differentiation into adipocyte was evaluated using mRNA expression value as an adipocyte marker. Total RNA was extracted from the cell suspension using RNeasy Mini kit (manufactured by QIAGEN) on seeding human pre-adipocytes in the above-mentioned medium composition (day 0) and days 4, 8 and 14. Using PrimeScript™ RT Master Mix (manufactured by Takara Bio Inc.), a reverse transcription reaction was performed on GeneAmp PCR System 9700 (manufactured by Applied Biosystems) to synthesize cDNA from total RNA. Each of the obtained cDNA samples diluted 1/10 with sterilized water was used as a PCR template. In addition, the dispensed and mixed cDNA was used as a sample for preparing an analytical curve. The analytical curve was set in a quantitative range from 1/3 to 1/243 dilution at a 3-fold common ratio. PCR was performed using each cDNA sample, analytical sample, Premix Ex Taq™ (manufactured by Takara Bio Inc.) and various TaqMan probes (manufactured by Applied Biosystems) and 7500 Real Time PCR System (manufactured by Applied Biosystems). The probes used (manufactured by Applied Biosystems) are shown in the following.
PPIA: HS99999904
PPAR gamma: HS011155134
LPL: HS00173425
FABP4: HS01086177

As a result, it was revealed that differentiation from human pre-adipocytes into adipocytes could be induced while maintaining cell survivability when differentiation into adipocytes was induced under the condition with addition of chitin nanofiber to the medium composition. The RLU value (ATP measurement, luminescence intensity) for cell survivability is shown in Table 6. The relative value when the value on day 0 is 100% is shown. The PPAR gamma, LPL and FABP4 mRNA expression relating to adipocyte differentiation evaluation are shown in Table 7, Table 8 and Table 9.

**[Table 6]**

| % | Day 4 | Day 8 | Day 14 |
|---|---|---|---|
| not added | 68.3 | 67.4 | 57.6 |
| chitin nanofiber | 95.9 | 102.5 | 93.0 |
| 0.01% | | | |

**[Table 7]**

| PPAR gamma | Day 0 | Day 4 | Day 8 | Day 14 |
|---|---|---|---|---|
| (PPIA corrected value) | | | | |
| not added | 0.15 | 1.19 | 2.11 | 2.16 |
| chitin nanofiber | 0.27 | 1.03 | 1.76 | 1.88 |
| 0.01% | | | | |

**[Table 8]**

| LPL | Day 0 | Day 4 | Day 8 | Day 14 |
|---|---|---|---|---|
| (PPIA corrected value) | | | | |
| not added | 0 | 1.32 | 7.03 | 9.48 |
| chitin nanofiber | 0 | 1.76 | 6.01 | 10.96 |
| 0.01% | | | | |

**[Table 9]**

| FABP4 | Day 0 | Day 4 | Day 8 | Day 14 |
|---|---|---|---|---|
| (PPIA corrected value) | | | | |
| not added | 0 | 0.83 | 1.99 | 1.85 |
| chitin nanofiber | 0 | 1.03 | 1.54 | 1.86 |
| 0.01% | | | | |

### (Experimental Example 6: Transportation test of human pre-adipocytes adhered to chitin nanofiber)

Chitin nanofiber (biomass nanofiber BiNFi-S 2 mass %, Sugino Machine Limited, Lot. GG30-G30) was suspended in ultrapure water (Milli-Q water) to 1% (w/v) and dispersed by stirring with heating at 90°C, and the aqueous solution (or suspension) was autoclave sterilized at 121°C for 20 min. A human pre-adipocyte proliferation medium (#CAS11K500, manufactured by TOYOBO CO., LTD.) or a medium composition obtained by adding a chitin nanofiber to a DMEM medium (#044-29765, manufactured by Wako Pure Chemical Industries, Ltd.) containing 10% FBS (#35-015-CV, manufactured by Corning Incorporated) (final concentration: 0.01% (w/v)), and a non-addition medium composition free of the chitin nanofiber were prepared. Successively, the cultured human pre-adipocytes (derived from subcutaneous tissue, #CAS02s05a, manufactured by TOYOBO CO., LTD.) were suspended in each of the above-mentioned medium compositions at 90000 cells/mL and seeded in a 6 well flat bottom ultra low attachment surface microplate (#3471, manufactured by Corning Incorporated) at 5 mL/well. The cells were cultured in a CO₂ incubator (37°C, 5% CO₂) in a static state for 3 days. After culturing, chitin nanofibers to which the human pre-adipocytes adhered were collected in a 50 mL tube. After centrifugation, a new human pre-adipocyte proliferation medium (#CAS11K500, manufactured by TOYOBO CO., LTD.) or a DMEM medium (#044-29765, manufactured by Wako Pure Chemical Industries, Ltd.) containing 10% FBS (#35-015-CV, manufactured by Corning Incorporated) was added to pelletized chitin nanofibers to which the human pre-adipocytes adhered or they were suspended therein, and transferred into a 6 well flat bottom ultra low attachment surface microplate (manufactured by Corning Incorporated, #3471) or a 15 mL tube. The 6 well flat bottom ultra low attachment surface microplate was cultured in a CO₂ incubator (37°C, 5% CO₂) in a static state for 7 days, and the 15 mL tube was stood in a 25°C incubator for 7 days.

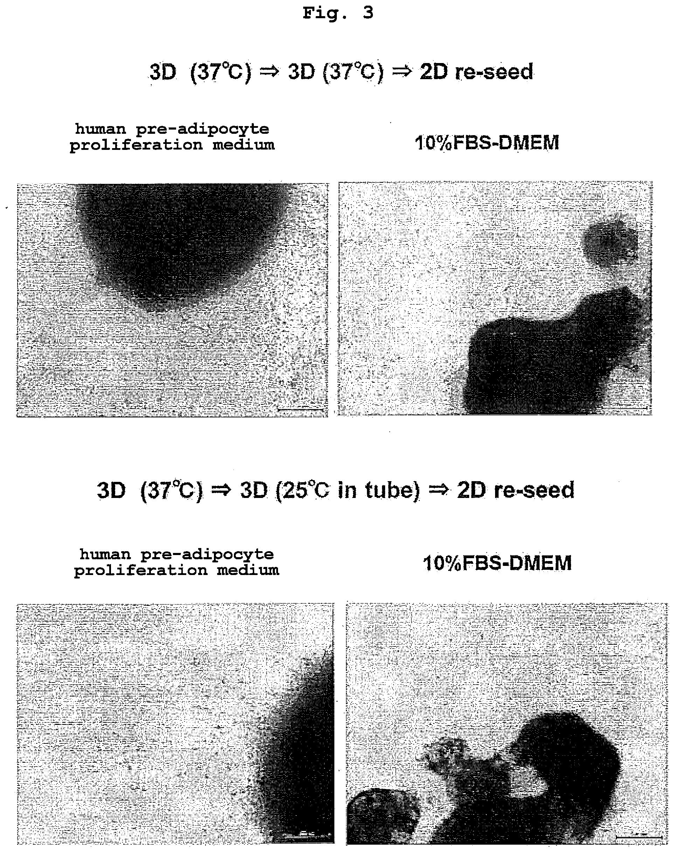

The human pre-adipocytes adhered to the chitin nanofiber which were maintained in a CO₂ incubator (37°C, 5% CO₂) or 25°C incubator for 7 days were re-seeded together with the medium in a 100 mm culture dish (#430167, manufactured by Corning Incorporated). The cells were further cultured for 7 days in a static state in a CO₂ incubator (37°C, 5% CO₂), and human pre-adipocytes migrated or proliferated from the chitin nanofiber were observed under a microscope (Fig. 3).

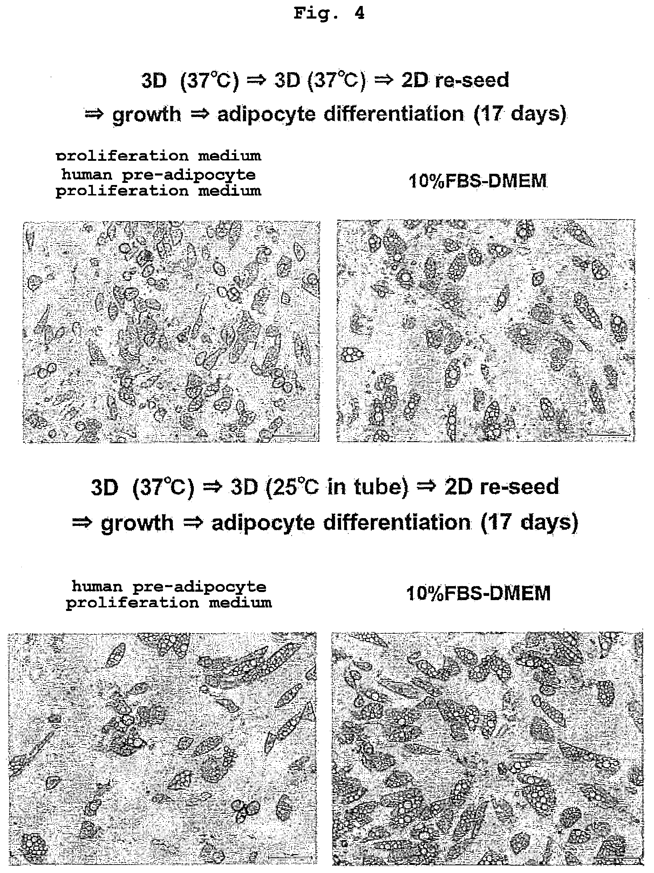

After confirmation with a microscope, the medium was removed, and human pre-adipocytes adhered to the dish and chitin nanofibers were washed with PBS(-) (#045-29795, manufactured by Wako Pure Chemical Industries, Ltd.), trypsin (#CA090K, manufactured by TOYOBO CO., LTD.) was added to recover human pre-adipocytes alone. The recovered human pre-adipocytes were suspended in a human pre-adipocyte proliferation medium (#CAS11K500, manufactured by TOYOBO CO., LTD.) or a DMEM medium (#044-29765, manufactured by Wako Pure Chemical Industries, Ltd.) containing 10% FBS (#35-015-CV, manufactured by Corning Incorporated), and seeded in a 6 well flat bottom ultra low attachment surface microplate (manufactured by Corning Incorporated, #3471) at 5 mL/well and cultured for 5 days. After culture for 5 days, the medium was changed to an adipocyte differentiation medium (#CA811D250, manufactured by TOYOBO CO., LTD.), culturing was further continued for 17 days, and differentiation induction into adipocyte was observed under a microscope (Fig. 4).

As a result, the human pre-adipocytes adhered to the chitin nanofiber showed migration activity from the chitin nanofibers and proliferation potency after standing for 7 days under both conditions of CO₂ incubator (37°C, 5% CO₂) and 25°C incubator (Fig. 3) and maintained differentiation potency into adipocytes (Fig. 4). Similar results were obtained using any of the human pre-adipocyte proliferation medium (#CAS11K500, manufactured by TOYOBO CO., LTD.) and the DMEM medium (#044-29765, manufactured by Wako Pure Chemical Industries, Ltd.) containing 10% FBS (#35-015-CV, manufactured by Corning Incorporated).

### (Experimental Example 7: Transportation test human adipose-derived mesenchymal stem cells adhered to the chitin nanofiber)

Chitin nanofiber (biomass nanofiber BiNFi-S 2 mass %, Sugino Machine Limited, Lot. GG30-G30) was suspended in ultrapure water (Milli-Q water) to 1% (w/v) and dispersed by stirring with heating at 90°C, and the aqueous solution (or suspension) was autoclave sterilized at 121°C for 20 min. An ADSC medium (#C-28009, manufactured by Takara Bio Inc.) or a medium composition obtained by adding a chitin nanofiber to a DMEM medium (#044-29765, manufactured by Wako Pure Chemical Industries, Ltd.) containing 10% FBS (#35-015-CV, manufactured by Corning Incorporated) (final concentration: 0.01% (w/v)), and a non-addition medium composition free of the chitin nanofiber were prepared. Successively, the cultured human adipose tissue-derived mesenchymal stem cells (#C-12977, manufactured by Takara Bio Inc.) were suspended in each of the above-mentioned medium compositions at 90000 cells/mL and seeded in a 6 well flat bottom ultra low attachment surface microplate (#3471, manufactured by Corning Incorporated) at 5 mL/well. The cells were cultured in a CO₂ incubator (37°C, 5% CO₂) in a static state for 3 days. After culturing, chitin nanofibers to which the human adipose-derived mesenchymal stem cells adhered were collected in a 50 mL tube. After centrifugation, a new ADSC medium (#C-28009, manufactured by Takara Bio Inc.) or a DMEM medium (#044-29765, manufactured by Wako Pure Chemical Industries, Ltd.) containing 10% FBS (#35-015-CV, manufactured by Corning Incorporated) was added to pelletized chitin nanofibers to which the human adipose-derived mesenchymal stem cells adhered, they were suspended therein, and transferred into a 6 well flat bottom ultra low attachment surface microplate (manufactured by Corning Incorporated, #3471) or a 15 mL tube. The 6 well flat bottom ultra low attachment surface microplate was incubated in a CO₂ incubator (37°C, 5% CO₂) in a static state for 7 days, and the 15 mL tube was stood in a 25°C incubator for 7 days.

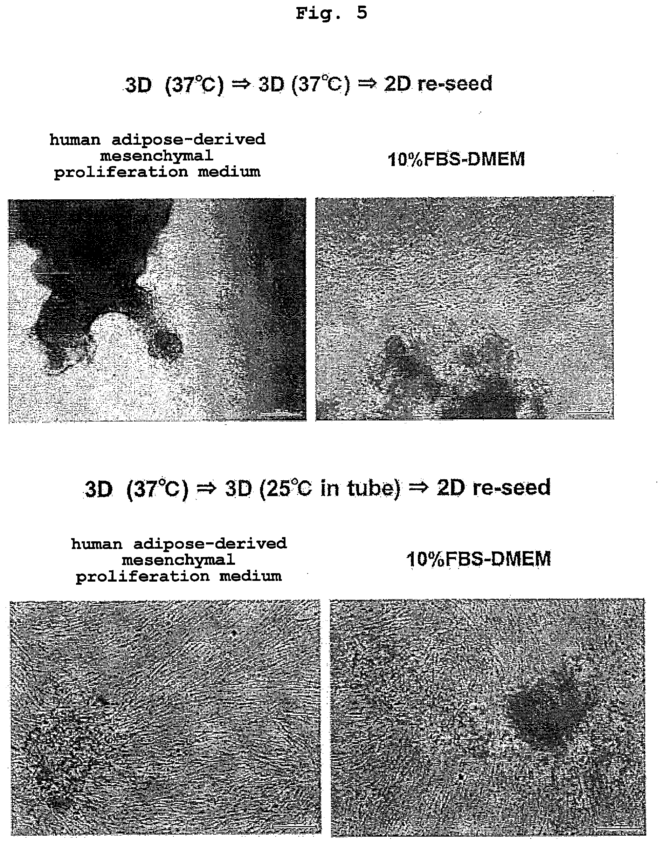

The human adipose-derived mesenchymal stem cells adhered to the chitin nanofiber which were maintained in a CO₂ incubator (37°C, 5% CO₂) or 25°C incubator for 7 days were re-seeded together with the medium in a 100 mm culture dish (#430167, manufactured by Corning Incorporated). The cells were further cultured for 7 days in a static state in a CO₂ incubator (37°C, 5% CO₂), and human adipose-derived mesenchymal stem cells migrated or proliferated from the chitin nanofiber were observed under a microscope (Fig. 5).

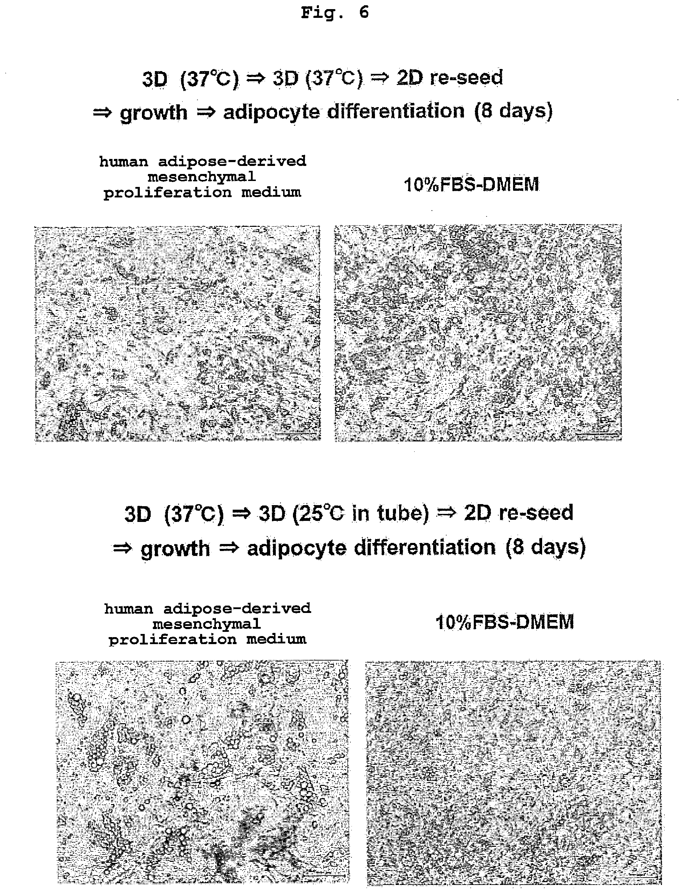

After confirmation with a microscope, the medium was removed, and human adipose-derived mesenchymal stem cells adhered to the dish and chitin nanofibers were washed with PBS(-) (#045-29795, manufactured by Wako Pure Chemical Industries, Ltd.), trypsin (#C-41210, manufactured by Takara Bio Inc.) was added to recover human adipose-derived mesenchymal stem cells alone. The recovered human adipose-derived mesenchymal stem cells were suspended in an ADSC medium (#C-28009, manufactured by Takara Bio Inc.) or a DMEM medium (#044-29765, manufactured by Wako Pure Chemical Industries, Ltd.) containing 10% FBS (#35-015-CV, manufactured by Corning Incorporated), and seeded in a 6 well flat bottom ultra low attachment surface microplate (manufactured by Corning Incorporated, #3471) at 5 mL/well and cultured for 2 days. After culture for 2 days, the medium was changed to an adipocyte differentiation medium (#C-28016, manufactured by Takara Bio Inc.), culturing was further continued for 8 days, and differentiation induction into adipocyte was observed under a microscope (Fig. 6).

As a result, the human adipose-derived mesenchymal stem cells adhered to the chitin nanofiber showed migration activity from the chitin nanofibers and proliferation potency after standing for 7 days under both conditions of CO₂ incubator (37°C, 5% CO₂) and 25°C incubator (Fig. 5) and maintained differentiation potency into adipocytes (Fig. 6). Similar results were obtained using any of the ADSC medium (#C-28009, manufactured by Takara Bio Inc.) and the DMEM medium (#044-29765, manufactured by Wako Pure Chemical Industries, Ltd.) containing 10% FBS (#35-015-CV, manufactured by Corning Incorporated).

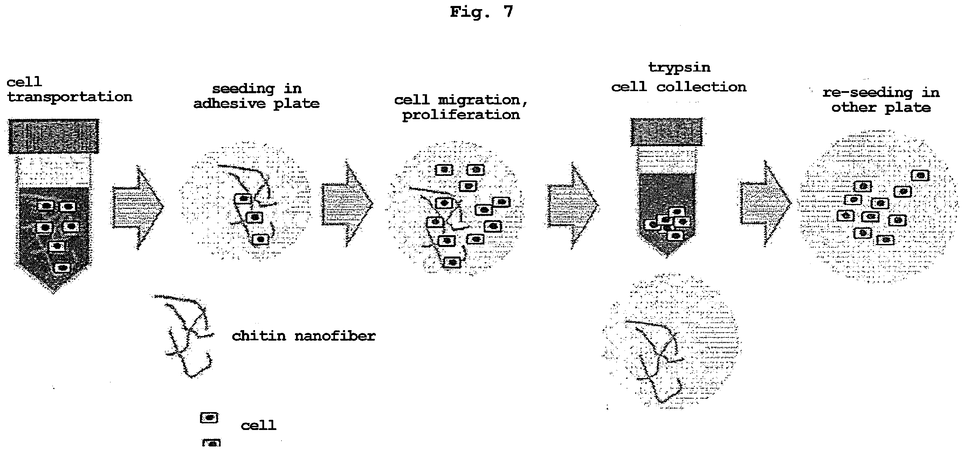

From the above results, a new method for transporting cells under non-freezing conditions, in which cells are transported while being attached to chitin nanofibers, the cells are directly re-seeded in a dish, the cells are migrated or proliferated on the dish, and collected with trypsin and the like was established. The model is shown in Fig. 7.

### (Experimental Example 8: MDCK cell proliferation effect by 3D culture using α chitin nanofiber, β chitin nanofiber, or chitosan nanofiber)

α chitin nanofiber (biomass nanofiber BiNFi-S 2 mass %, Sugino Machine Limited) prepared according to WO 2015/111686 A1 was suspended in ultrapure water (Milli-Q water) to 1% (w/v), dispersed by stirring with heating at 90°C, and the aqueous solution was autoclave sterilized at 121°C for 20 min. Furthermore, chitosan nanofiber and β chitin nanofiber were suspended in ultrapure water (Milli-Q water) to 1% (w/v), dissolved by stirring with heating at 90°C, and the aqueous solution was autoclave sterilized at 121°C for 20 min. The nanofibers used were α chitin nanofiber nanofibrillated under fibrillating condition of 200 MPa, pass 10 times (sample 1), α chitin nanofiber nanofibrillated under fibrillating condition of 200 MPa, pass 1 time (sample 2), chitosan nanofiber nanofibrillated under fibrillating condition of 200 MPa, pass 10 times (sample 3), and β chitin nanofiber nanofibrillated under fibrillating condition of 200 MPa, pass 10 times (sample 4) .

Medium compositions were prepared by adding each of the nanofiber samples (1 - 4) at a final concentration of 0.03% (w/v) to serum-free medium KBM220 medium (manufactured by KOHJIN BIO). Successively, the cultured canine kidney renal tubule epithelial cell line MDCK (manufactured by DS PHARMA BIOMEDICAL CO., LTD.) was seeded in a medium composition added with each of the above-mentioned nanofiber samples (1 - 4) at 66666 cells/mL and dispensed in a 125 mL flask (Thermo Scientific, 4115-0125) by 30 mL. Each flask was continuously cultured in a shaken state (cfg50 rpm) in a CO₂ incubator (37°C, 5% CO₂) for 6 days. On day 6, MDCK cells adhered to each nanofiber sample (1 - 4) and the nanofibers were recovered in a 50 mL tube, centrifuged (cfg 1000 rpm, 3 min) and the medium alone was removed. A new KBM220 medium was added and suspended in each of the remaining nanofiber samples and MDCK cells, and the suspension was placed back in the original 125 mL flask and continuously cultured in a shake state (cfg50 rpm). The following operation for medium change was also performed on day 9 and day 13.

The culture media on days 0, 6, 13, 17 were suspended by pipetting, an ATP reagent (100 µL, CellTiter-Glo™ Luminescent Cell Viability Assay, manufactured by Promega) was added and reacted per 100 µL of the cell suspension and the mixture was stood for about 10 min at room temperature. The luminescence intensity (RLU value) was measured by FlexStation3 (manufactured by Molecular Devices) and the luminescence value of the medium alone was subtracted to measure the number of viable cells as a mean of 4 samples.

As a result, when MDCK cells were cultured in a medium composition containing each nanofiber sample (1 - 4) in a 125 mL flask, the highest cell proliferation promoting action was found in sample 2 with addition of α chitin nanofiber. A cell proliferation promoting effect was also found in the α chitin nanofiber of sample 1 and the chitosan nanofiber of sample 3. On the other hand, the β chitin nanofiber of sample 4 showed a weak cell proliferation promoting action. The RLU value (ATP measurement, luminescence intensity) in each culture is shown in Table 10.

**[Table 10]**

| sample | nanofiber | Day 0 | Day 6 | Day 13 | Day 17 |
|---|---|---|---|---|---|
| 1 | α chitin (sample 1) | 7307 | 10351 | 18945 | 25889 |
| 2 | α chitin (sample 2) | 6985 | 17469 | 38108 | 47912 |
| 3 | chitosan (sample 3) | 6905 | 12058 | 21853 | 25649 |
| 4 | β chitin (sample 4) | 6566 | 10663 | 10093 | 9916 |

### (Experimental Example 9: MDCK cell proliferation effect by 3D culture using α chitin nanofiber under each fibrillating condition)

α chitin nanofiber (biomass nanofiber BiNFi-S 2 mass %, Sugino Machine Limited) prepared according to WO 2015/111686 A1 was suspended in ultrapure water (Milli-Q water) to 1% (w/v), dispersed by stirring with heating at 90°C, and the aqueous solution was autoclave sterilized at 121°C for 20 min. The α chitin nanofibers used were α chitin nanofiber nanofibrillated under condition of 200 MPa, pass 10 times (sample 1), α chitin nanofiber nanofibrillated under condition of 200 MPa, pass 1 time (sample 2), α chitin nanofiber nanofibrillated under condition of 100 MPa, pass 1 time (sample 5), α chitin nanofiber nanofibrillated under condition of 200 MPa, pass 3 times (sample 6), α chitin nanofiber nanofibrillated under condition of 100 MPa, pass 5 times (sample 7), and α chitin material (sample 8).

Medium compositions were prepared by adding each of the nanofiber samples (1, 2, 5 - 8) at a final concentration of 0.03% (w/v) to serum-free medium KBM220 medium (manufactured by KOHJIN BIO) and a non-addition medium composition free of the above-mentioned substrate (sample 9) was prepared. Successively, canine kidney renal tubule epithelial cell line MDCK (manufactured by DS PHARMA BIOMEDICAL CO., LTD.) was seeded in a medium composition added with each of the above-mentioned nanofiber samples (1, 2, 5 - 8) at 66666 cells/mL and dispensed in a 125 mL flask (Thermo Scientific, 4115-0125) by 30 mL. Each flask was continuously cultured in a shaken state (cfg50 rpm) in a CO₂ incubator (37°C, 5% CO₂) for 7 days.

The culture media on days 0, 3, 7 were suspended by pipetting, an ATP reagent (100 µL, CellTiter-Glo™ Luminescent Cell Viability Assay, manufactured by Promega) was added and reacted per 100 µL of the cell suspension and the mixture was stood for about 10 min at room temperature. The luminescence intensity (RLU value) was measured by FlexStation3 (manufactured by Molecular Devices) and the luminescence value of the medium alone was subtracted to measure the number of viable cells as a mean of 4 samples.

As a result, when MDCK cells were cultured in a medium composition containing each nanofiber sample (1, 2, 5 - 8) in a 125 mL flask, the highest cell proliferation promoting action was found in sample 5 and sample 6 by the addition of α chitin nanofiber with low fibrillation. On the other hand, a cell proliferation promoting action of α chitin material of sample 8was not found. The RLU value (ATP measurement, luminescence intensity) in each culture is shown in Table 11.

**[Table 11]**

| sample | nanofiber (fibrillation) | Day 0 | Day 3 | Day 7 |
|---|---|---|---|---|
| 1 | α chitin (sample 1) | 7884 | 17483 | 27007 |
| 2 | α chitin (sample 2) | 8146 | 32372 | 42390 |
| 5 | α chitin (sample 5) | 8058 | 23258 | 35941 |
| 6 | α chitin (sample 6) | 8328 | 23941 | 50496 |
| 7 | α chitin (sample 7) | 8143 | 29171 | 55898 |
| 8 | α chitin material (sample 8) | 7619 | 2401 | 4507 |
| 9 | not added (sample 9) | 8096 | 5374 | 2947 |

### (Experimental Example 10: Human adipose-derived mesenchymal stem cell proliferation action by 3D culture using α chitin nanofiber under each fibrillating condition)

α chitin nanofiber (biomass nanofiber BiNFi-S 2 mass %, Sugino Machine Limited) prepared according to WO 2015/111686 A1 was suspended in ultrapure water (Milli-Q water) to 1% (w/v), dispersed by stirring with heating at 90°C, and the aqueous solution was autoclave sterilized at 121°C for 20 min. The α chitin nanofibers used were α chitin nanofiber nanofibrillated under condition of 200 MPa, pass 10 times (sample 1), α chitin nanofiber nanofibrillated under condition of 200 MPa, pass 1 time (sample 2), and chitosan nanofiber nanofibrillated under condition of 200 MPa, pass 5 times (sample 10).

Medium compositions were prepared by adding each of the nanofiber samples (1, 2, 10) at a final concentration of 0.001% (w/v), 0.003%, or 0.01% to mesenchymal stem cell proliferation medium 2 medium (manufactured by Takara Bio Inc.) and a non-addition medium composition free of the above-mentioned substrate (sample 9) was prepared. Successively, the cultured human bone marrow-derived mesenchymal stem cells (C-12974, manufactured by Takara Bio Inc.) was seeded in the above-mentioned chitin nanofiber-added medium compositions or non-addition medium composition at 20000 cells/mL and dispensed in the wells of a 96 well flat bottom ultra low attachment surface microplate (manufactured by Corning Incorporated, #3474) at 150 µL per well. Each plate was continuously cultured in a static state in a CO₂ incubator (37°C, 5% CO₂) for 10 days. An ATP reagent (150 µL, CellTiter-Glo™ Luminescent Cell Viability Assay, manufactured by Promega) was added to the culture media on days 3, 7, 10 and the mixtures were suspended and stood for about 10 min at room temperature. The luminescence intensity (RLU value) was measured by FlexStation3 (manufactured by Molecular Devices) and the luminescence value of the medium alone was subtracted to measure the number of viable cells as a mean of 4 samples.

As a result, when human bone marrow mesenchymal cells were cultured in a medium composition containing each nanofiber sample (1, 2, 10) in a 96 well flat bottom ultra low attachment surface microplate, the highest cell proliferation promoting action was found in sample 2 and sample 10 as compared to sample 1 by the addition of α chitin nanofiber with low fibrillation. The RLU value (ATP measurement, luminescence intensity) in each culture is shown in Table 12.

**[Table 12]**

| | Day 0 | Day 3 | Day 7 | Day 10 |
|---|---|---|---|---|
| not added (sample 9) | 4067 | 4178 | 2875 | 4391 |
| sample 1 | 4664 | 5752 | 3790 | 6212 |
| 0.001% | | | | |
| sample 1 | 4516 | 5366 | 5865 | 7210 |
| 0.003% | | | | |
| sample 1 | 4125 | 6005 | 7964 | 10299 |
| 0.01% | | | | |
| sample 2 | 4092 | 5094 | 6877 | 7319 |
| 0.001% | | | | |
| sample 2 | 4193 | 5499 | 9676 | 13407 |
| 0.003% | | | | |
| sample 2 | 3872 | 5564 | 8894 | 15736 |
| 0.01% | | | | |
| sample 10 | 4029 | 4769 | 6820 | 7898 |
| 0.001% | | | | |
| sample 10 | 4494 | 6419 | 11462 | 14588 |
| 0.003% | | | | |
| sample 10 | 3249 | 5204 | 10485 | 15105 |
| 0.01% | | | | |

### (Experimental Example 11: MDCK cell proliferation action by 3D culture using α chitin nanofiber under high fibrillating condition)

α chitin nanofiber (biomass nanofiber BiNFi-S 2 mass %, Sugino Machine Limited) prepared according to WO 2015/111686 A1 was suspended in ultrapure water (Milli-Q water) to 1% (w/v), dispersed by stirring with heating at 90°C, and the aqueous solution was autoclave sterilized at 121°C for 20 min. The α chitin nanofibers used were α chitin nanofiber nanofibrillated under condition of 200 MPa, pass 1 time (sample 2), chitosan nanofiber nanofibrillated under condition of 200 MPa, pass 20 times (sample 11), α chitin nanofiber nanofibrillated under condition of 200 MPa, pass 50 times (sample 12), α chitin nanofiber nanofibrillated under condition of 200 MPa, pass 100 times (sample 13), and α chitin nanofiber nanofibrillated under condition of 200 MPa, pass 200 times (sample 14).

Medium compositions were prepared by adding each of the nanofiber samples (2, 11 - 14) at a final concentration of 0.006% (w/v), 0.02%, or 0.06% to serum-free medium KBM220 medium (manufactured by KOHJIN BIO) and a non-addition medium composition free of the above-mentioned substrate (sample 9) was prepared. Successively, canine kidney renal tubule epithelial cell line MDCK (manufactured by DS PHARMA BIOMEDICAL CO., LTD.) was seeded in the above-mentioned chitin nanofiber-added medium compositions or non-addition medium composition at 33333 cells/mL and dispensed in the wells of a 96 well flat bottom ultra low attachment surface microplate (manufactured by Corning Incorporated, #3474) at 150 µL per well. Each plate was continuously cultured in a static state in a CO₂ incubator (37°C, 5% CO₂) for 6 days. An ATP reagent (100 µL, CellTiter-Glo™ Luminescent Cell Viability Assay, manufactured by Promega) was added to the culture media on days 3, 6 and the mixtures were suspended and stood for about 10 min at room temperature. The luminescence intensity (RLU value) was measured by FlexStation3 (manufactured by Molecular Devices) and the luminescence value of the medium alone was subtracted to measure the number of viable cells as a mean of 4 samples.

As a result, when MDCK cells were cultured in a medium composition containing each nanofiber sample (2, 11-14) in a 96 well flat bottom ultra low attachment surface microplate, a weak cell proliferation promoting action was found in sample 11 as compared to sample 2 by the addition of α chitin nanofiber with high fibrillation. Samples (12-14) with increased fibrillation showed only a weaker promoting action. The RLU value (ATP measurement, luminescence intensity) in each culture is shown in Table 13.

**[Table 13]**

| | Day 3 | Day 6 |
|---|---|---|
| not added (sample 9) | 7241 | 8361 |
| sample 2 (0.006%) | 17882 | 47095 |
| sample 2 (0.02%) | 16804 | 53055 |
| sample 2 (0.06%) | 16134 | 56368 |
| sample 11 (0.006%) | 14312 | 36919 |
| sample 11 (0.02%) | 15382 | 39506 |
| sample 11 (0.06%) | 14744 | 41891 |
| sample 12 (0.006%) | 13896 | 28671 |
| sample 12 (0.02%) | 14692 | 31694 |
| sample 12 (0.06%) | 15321 | 36274 |
| sample 13 (0.006%) | 13889 | 29671 |
| sample 13 (0.02%) | 13754 | 30630 |
| sample 13 (0.06%) | 15345 | 32912 |
| sample 14 (0.006%) | 13509 | 27234 |
| sample 14 (0.02%) | 14106 | 33868 |
| sample 14 (0.06%) | 14106 | 33868 |

### (Experimental Example 12: Preparation of sheet of human pre-adipocytes adhered to chitin nanofiber)

Chitin nanofiber (biomass nanofiber BiNFi-S 2 mass %, Sugino Machine Limited, Lot. GG30-G30) prepared in Production Example 1 was suspended in ultrapure water (Milli-Q water) to 1% (w/v), dispersed by stirring with heating at 90°C, and the aqueous solution was autoclave sterilized at 121°C for 20 min. A medium composition was prepared by adding a chitin nanofiber at a final concentration of 0.01% (w/v) to human pre-adipocyte proliferation medium (#CAS11K500, manufactured by TOYOBO CO., LTD.) and a non-addition medium composition free of the above-mentioned substrate was prepared. Successively, the cultured human pre-adipocytes (derived from subcutaneous tissue, #CAS02s05a, manufactured by TOYOBO CO., LTD.) was seeded in the above-mentioned chitin nanofiber-added medium composition or non-addition medium composition at 33333 cells/mL and dispensed in the wells of a 96 well flat bottom ultra low attachment surface microplate (manufactured by Corning Incorporated, #3474) at 150 µL per well. Each plate was continuously cultured in a static state in a CO₂ incubator (37°C, 5% CO₂) for 10 days. On day 10, human pre-adipocytes were observed and photographed.

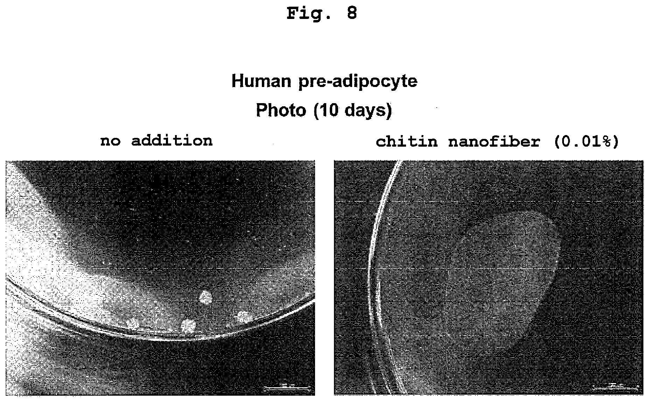

As a result, it was clarified that when human pre-adipocytes are cultured for 10 days on a 96 well flat bottom ultra low attachment surface microplate by using a chitin nanofiber which is the medium composition of the present invention, a cell sheet is obtained by fusion of the chitin nanofiber and the human pre-adipocytes as shown in Fig. 8.

### (Experimental Example 13: Proliferation of human adipose tissue-derived mesenchymal stem cells by 3D culture using chitin nanofiber)

α chitin nanofiber (biomass nanofiber BiNFi-S 2 mass %, Sugino Machine Limited) prepared according to WO 2015/111686 A1 was suspended in ultrapure water (Milli-Q water) to 1% (w/v), dispersed by stirring with heating at 90°C, and the aqueous solution was autoclave sterilized at 121°C for 20 min. The α chitin nanofibers used were α chitin nanofiber nanofibrillated under condition of 200 MPa, pass 5 times (sample 15), α chitin nanofiber nanofibrillated under condition of 200 MPa, pass 20 times (sample 11), α chitin nanofiber nanofibrillated under condition of 200 MPa, pass 50 times (sample 12), α chitin nanofiber nanofibrillated under condition of 200 MPa, pass 100 times (sample 13), and α chitin nanofiber nanofibrillated under condition of 200 MPa, pass 200 times (sample 14). A medium composition obtained by adding a chitin nanofiber to a mesenchymal stem cell proliferation medium (C-28009, manufactured by Takara Bio Inc.) which is a low serum medium (final concentration: 0.002% (w/v), 0.006% (w/v), 0.02%), and a non-addition medium composition free of the above-mentioned substrate (sample 9) was prepared. Successively, the cultured human adipose-derived mesenchymal stem cells (C-12977, manufactured by Takara Bio Inc.) were suspended in each of the above-mentioned medium compositions at 13333 cells/mL, and seeded in a 96 well flat bottom ultra low attachment surface microplate (manufactured by Corning Incorporated, #3474) at 150 µL/well. The cells were cultured in a CO₂ incubator (37°C, 5% CO₂) in a static state for 10 days. An ATP reagent (150 µL, CellTiter-Glo™ Luminescent Cell Viability Assay, manufactured by Promega) was added to the culture medium on days 4, 8, 11 and suspended and the mixture was stood for about 10 min at room temperature. The luminescence intensity (RLU value) was measured by FlexStation3 (manufactured by Molecular Devices) and the luminescence value of the medium alone was subtracted to calculate the number (mean of 4 samples) of viable cells.

As a result, when human adipose-derived mesenchymal stem cells were cultured in a medium composition containing each nanofiber sample (15, 11-14) in a 96 well flat bottom ultra low attachment surface microplate, a weak cell proliferation promoting action as compared to sample 15 was found by the addition of α chitin nanofiber with high fibrillation (sample 11). Samples (12-14) with increased fibrillation did not show a clear proliferation promoting action. The RLU values (ATP measurement, luminescence intensity) in respective cultures are shown in Table 14 and Table 15.

**[Table 14]**

| | Day 4 | Day 8 | Day 11 |
|---|---|---|---|
| not added (sample 9) | 4972 | 8512 | 6938 |
| sample 15 (0.002%) | 10481 | 18950 | 19409 |
| sample 15 (0.006%) | 9906 | 17758 | 23314 |
| sample 15 (0.02%) | 9861 | 16065 | 21629 |
| sample 11 (0.002%) | 9170 | 13776 | 14939 |
| sample 11 (0.006%) | 9684 | 13790 | 18219 |
| sample 11 (0.02%) | 9250 | 12270 | 16744 |
| sample 12 (0.002%) | 8491 | 10658 | 11137 |
| sample 12 (0.006%) | 8083 | 8902 | 10370 |
| sample 12 (0.02%) | 7706 | 7436 | 8271 |

**[Table 15]**

| | Day 4 | Day 8 | Day 11 |
|---|---|---|---|
| not added (sample 9) | 4648 | 3960 | 5375 |
| sample 15 (0.002%) | 10298 | 18656 | 16750 |
| sample 15 (0.006%) | 10042 | 17958 | 21934 |
| sample 15 (0.02%) | 10020 | 15931 | 24792 |
| sample 13 (0.002%) | 8765 | 10499 | 12185 |
| sample 13 (0.006%) | 8631 | 9168 | 11430 |
| sample 13 (0.02%) | 7341 | 7715 | 8381 |
| sample 14 (0.002%) | 8258 | 11109 | 12561 |
| sample 14 (0.006%) | 8504 | 9727 | 10712 |
| sample 14 (0.02%) | 7500 | 7327 | 8287 |

The property values (measured concentration, viscosity, median particle size and average particle size of α chitin) of the α chitin dispersions prepared under various fibrilating conditions (pressure, grinding number) used in Experimental Examples 8 - 13 are shown in Table 16.

**[Table 16]**

| Pressure MPa | grinding number Pass | measured concentration % (w/v) | viscosity mPa·S (22.3-26.2°C) | laser diffraction median particle size µm | laser diffraction average particle size µm |
|---|---|---|---|---|---|
| 200 | 1 | 0.77% | 1.02 | 55.9 | 80 |
| | | 0.99% | 2.10 | 58.5 | 68.6 |
| 200 | 3 | 1.15% | 4.23 | 104.6 | 132.2 |
| 200 | 5 | 1.19% | 6.75 | 68.3 | 83.5 |
| | | 1.05% | 6.73 | 65 | 80.7 |
| 200 | 10 | 1.17% | 71.90 | 29.8 | 40.8 |
| 200 | 20 | 0.96% | 58.30 | 38.2 | 52.8 |
| | | 0.96% | 53.00 | 34.3 | 40.8 |
| 200 | 50 | 1.05% | 82.10 | 0.6 | 0.93 |
| 200 | 100 | 1.12% | 58.50 | 0.53 | 0.8 |
| 200 | 200 | 1.14% | 32.70 | 0.67 | 0.84 |
| 100 | 5 | 0.84% | 8.37 | 96.2 | 116.1 |

### [Industrial Applicability]

Nanofibers composed of water-insoluble polysaccharides can be used as a common carrier in various operations such as i) culture, ii) differentiation induction, iii) transportation and preservation under non-freezing conditions, iv) transplantation, v) recovery of bioactive substance from culture supernatant and the like of adherent cells such as mesenchymal stem cells, pre-adipocytes and the like. Using the carrier of the present invention, therefore, plural operations selected from the aforementioned i) culture, ii) differentiation induction, iii) transportation and preservation under non-freezing conditions, iv) transplantation, v) recovery of bioactive substance from culture supernatant can be performed continuously.

The contents disclosed in any publication cited herein, including patents and patent applications, are hereby incorporated in their entireties by reference, to the extent that they have been disclosed herein.

### (Indication of related application)

This application is based on patent application Nos. 2017-68377 filed in Japan (filing date: March 30, 2017) and 2017-174237 filed in Japan (filing date: September 11, 2017), the contents of which are incorporated in full herein by reference.

## Claims

1. A carrier comprising a nanofiber composed of water-insoluble polysaccharides, for continuously subjecting adherent cells to two or more treatments selected from the group consisting of the following (1) to (3):
(1) suspension culture,
(2) preservation and/or transportation under non-freezing conditions, and
(3) transplantation.

2. The carrier according to claim 1 wherein the suspension culture is for maintenance or proliferation of adherent cells, differentiation induction of adherent cells and/or production of humoral factor by adherent cells.

3. The carrier according to claim 1 or 2 wherein the carrier is for continuously subjecting the adherent cells to the treatments (1) to (3).

4. The carrier according to any one of claims 1 to 3 wherein the adherent cell is a stem cell or a progenitor cell.

5. The carrier according to any one of claims 1 to 3 wherein the adherent cell is a mesenchymal stem cell or a pre-adipocyte.

6. The carrier according to any one of claims 1 to 5 wherein the water-insoluble polysaccharide is chitin or chitosan.

7. A method for treating an adherent cell comprising continuously subjecting the adherent cell to two or more treatments selected from the group consisting of the following (1) to (3) while being attached to a nanofiber composed of water-insoluble polysaccharides:
(1) suspension culture,
(2) preservation and/or transportation under non-freezing conditions, and
(3) transplantation.

8. The method according to claim 7 wherein the suspension culture is for maintenance or proliferation of adherent cells, differentiation induction of adherent cells and/or production of humoral factor by adherent cells.

9. The method according to claim 7 or 8 wherein the adherent cells are continuously subjected to the treatments (1) to (3).

10. The method according to any one of claims 7 to 9 wherein the adherent cell is a stem cell or a progenitor cell.

11. The method according to any one of claims 7 to 9 wherein the adherent cell is a mesenchymal stem cell or a pre-adipocyte.

12. The method according to any one of claims 7 to 11 wherein the water-insoluble polysaccharide is chitin or chitosan.

13. The method according to any one of claims 7 to 12 further comprising treating the adherent cells attached to the nanofiber composed of water-insoluble polysaccharides with a degrading enzyme of the water-insoluble polysaccharides to degrade the nanofiber, detaching the adherent cells from the nanofiber and recovering the adherent cells.

14. A method for recovering an adherent cell attached to a nanofiber composed of water-insoluble polysaccharides comprising treating the adherent cell with a degrading enzyme of the water-insoluble polysaccharides to degrade the nanofiber, detaching the adherent cell from the nanofiber and recovering the adherent cell.

15. The method according to claim 14 wherein the water-insoluble polysaccharide is chitin or chitosan.

16. The method according to claim 15 wherein the degrading enzyme is chitinase, chitobiase, chitosanase or β-1,3-glucanase.
